# EUROPÄISCHE PATENTSCHRIFT

(11) **EP 1 360 284 B1**
(45) Veröffentlichungstag und Bekanntmachung des Hinweises auf die Patenterteilung: **16.07.2008**
(21) Anmeldenummer: 02750504.9
(22) Anmeldetag: 08.02.2002
(51) Int. Cl.: C12N 9/58, C07K 1/113, C12N 15/70

(54) **REKOMBINANTE PROTEINASE K**
RECOMBINANT PROTEINASE K
PROTEINASE K RECOMBINANTE

(30) Priorität: 09.02.2001 DE 10105912
(43) Veröffentlichungstag der Anmeldung: 12.11.2003
(73) Patentinhaber: Roche Diagnostics GmbH, 68305 Mannheim (DE); F. Hoffmann-La Roche AG, 4070 Basel (CH)
(72) Erfinder: MUELLER, Rainer, 82377 Penzberg (DE); THALHOFER, Johann-Peter, 82362 Weilheim (DE); REXER, Bernhard, 82362 Weilheim (DE); SCHMUCK, Rainer, 83671 Benediktbeuern (DE); GEIPEL, Frank, 82377 Penzberg (DE); GLASER, Stephan, 82402 Seeshaupt (DE); SCHOEN, Helmut, 82377 Penzberg (DE); MEIER, Thomas, 81369 Muenchen (DE); RUDOLPH, Rainer, 06120 Halle (DE); LILIE, Hauke, 06112 Halle (DE); SCHOTT, Bjoern, 35390 Giessen (DE)
(86) Internationale Anmeldenummer: PCT/EP2002/001322
(87) Internationale Veröffentlichungsnummer: WO 2002/072634

(56) Entgegenhaltungen:
- EP-A- 0 396 106
- WO-A-87/02673
- WO-A-88/07581
- WO-A-96/28556
- DE-A- 1 965 281
- CREIGHTON TE: "Protein Function - A practical approach" 1997 , OXFORD UNIVERSITY PRESS XP001106186 in der Anmeldung erwähnt * Chapter 3, Rudolph, R. "Folding proteins" Seite 75 -Seite 82; Tabelle 1
- DE BERNARDEX CLARK ELIANA ET AL: "Inhibition of aggregation side reactions during in vitro protein folding." METHODS IN ENZYMOLOGY, Bd. 309, 1999, Seiten 217-236, XP001106196 ISBN: 0-12-182210-9 in der Anmeldung erwähnt
- KANE J F: "EFFECTS OF RARE CODON CLUSTERS ON HIGH-LEVEL EXPRESSION OF HETEROLOGOUS PROTEINS IN ESCHERICHIA COLI" CURRENT OPINION IN BIOTECHNOLOGY, LONDON, GB, Bd. 6, Nr. 5, Oktober 1995 (1995-10), Seiten 494-500, XP002926790 ISSN: 0958-1669 in der Anmeldung erwähnt
- PAIN R.H. (ED): "Mechanisms of Protein Folding - Frontiers in Molecular Biology" 1994 , OXFORD UNIVERSITY PRESS XP002215954 ISBN: 0-19-963397-5 * Chapter 9, "Protein Folding in Biotechnology"
- GUNKEL F A ET AL: "PROTEINASE K FROM TRITIRACHIUM-ALBUM LIMBER CHARACTERIZATION OF THE CHROMOSOMAL GENE AND EXPRESSION OF THE COMPLEMENTARY DNA IN ESCHERICHIA-COLI" EUROPEAN JOURNAL OF BIOCHEMISTRY, Bd. 179, Nr. 1, 1989, Seiten 185-194, XP001106323 ISSN: 0014-2956

### SEQUENCE LISTING

<110> Roche Diagnostics GmbH
<120> Rekombinante Proteinase K
<130> 5388/00/DE
<140>
   <141>
<160> 24
<170> PatentIn Ver. 2.1
   <210> 1
   <211> 384
   <212> PRT
   <213> Tritirachium album limber
<400> 1
   Met Arg Leu Ser Val Leu Leu Ser Leu Leu Pro Leu Ala Leu Gly Ala
    1               5                  10                  15
   Pro Ala Val Glu Gln Arg Ser Glu Ala Ala Pro Leu Ile Glu Ala Arg
               20                  25                  30
   Gly Glu Met Val Ala Asn Lys Tyr Ile Val Lys Phe Lys Glu Gly Ser
           35                  40                  45
   Ala Leu Ser Ala Leu Asp Ala Ala Met Glu Lys Ile Ser Gly Lys Pro
       50                  55                  60
   Asp His Val Tyr Lys Asn Val Phe Ser Gly Phe Ala Ala Thr Leu Asp
   65                  70                  75                  80
   Glu Asn Met Val Arg Val Leu Arg Ala His Pro Asp Val Glu Tyr Ile
                   85                  90                  95
   Glu Gln Asp Ala Val Val Thr Ile Asn Ala Ala Gln Thr Asn Ala Pro
               100                 105                 110
   Trp Gly Leu Ala Arg Ile Ser Ser Thr Ser Pro Gly Thr Ser Thr Tyr
           115                 120                 125
   Tyr Tyr Asp Glu Ser Ala Gly Gln Gly Ser Cys Val Tyr Val Ile Asp
       130                 135                 140
   Thr Gly Ile Glu Ala Ser His Pro Glu Phe Glu Gly Arg Ala Gln Met
   145                 150                 155                 160
   Val Lys Thr Tyr Tyr Tyr Ser Ser Arg Asp Gly Asn Gly His Gly Thr
                   165                 170                 175
   His Cys Ala Gly Thr Val Gly Ser Arg Thr Tyr Gly Val Ala Lys Lys
               180                 185                 190
   Thr Gln Leu Phe Gly Val Lys Val Leu Asp Asp Asn Gly Ser Gly Gln
           195                 200                 205
   Tyr Ser Thr Ile Ile Ala Gly Met Asp Phe Val Ala Ser Asp Lys Asn
       210                 215                 220
   Asn Arg Asn Cys Pro Lys Gly Val Val Ala Ser Leu Ser Leu Gly Gly
   225                 230                 235                 240
   Gly Tyr Ser Ser Ser Val Asn Ser Ala Ala Ala Arg Leu Gln Ser Ser
                   245                 250                 255
   Gly Val Met Val Ala Val Ala Ala Gly Asn Asn Asn Ala Asp Ala Arg
               260                 265                 270
   Asn Tyr Ser Pro Ala Ser Glu Pro Ser Val Cys Thr Val Gly Ala Ser
           275                 280                 285
   Asp Arg Tyr Asp Arg Arg Ser Ser Phe Ser Asn Tyr Gly Ser Val Leu
       290                 295                 300
   Asp Ile Phe Gly Pro Gly Thr Ser Ile Leu Ser Thr Trp Ile Gly Gly
   305                 310                 315                 320
   Ser Thr Arg Ser Ile Ser Gly Thr Ser Met Ala Thr Pro His Val Ala
                   325                 330                 335
   Gly Leu Ala Ala Tyr Leu Met Thr Leu Gly Lys Thr Thr Ala Ala Ser
               340                 345                 350
   Ala Cys Arg Tyr Ile Ala Asp Thr Ala Asn Lys Gly Asp Leu Ser Asn
           355                 360                 365
   Ile Pro Phe Gly Thr Val Asn Leu Leu Ala Tyr Asn Asn Tyr Gln Ala
       370                 375                 380
<210> 2
   <211> 1116
   <212> DNA
   <213> Tritirachium album limber
<400> 2
   atggctcctg ccgttgagca gcgctccgag gctgctcctc tgatcgaggc ccgcggcgag 60
   atggttgcca acaagtacat cgtcaagttc aaggagggta gcgctctttc cgctctggat 120
   gctgccatgg agaagatctc tggcaagccc gaccacgtct acaagaacgt cttcagcggt 180
   ttcgctgcga ccctggacga gaacatggtt cgggttctcc gcgcccaccc cgatgttgag 240
   tacatcgagc aggatgctgt tgtcaccatc aacgctgcgc agaccaacgc tccctggggc 300
   ctggctcgca tctccagcac cagccccggt acctctacct actactatga cgaatctgcc 360
   ggccaaggct cctgcgtcta cgtgatcgac accggtatcg aggcatcgca ccccgagttc 420
   gagggtcgtg cccagatggt caagacctac tactactcca gtcgcgacgg taacggtcac 480
   ggcacccact gcgctggtac cgttggctcc cgtacctacg gtgtcgccaa gaagacccag 540
   ctgttcggtg tcaaggtcct ggatgacaac ggcagtggcc agtactccac catcatcgcc 600
   ggtatggact tcgttgccag cgacaagaac aaccgcaact gccccaaagg tgtcgttgcc 660
   tccttatccc tgggcggtgg ttactcctcc tccgtgaaca gcgccgctgc ccgcctccag 720
   agctctggtg tcatggtcgc cgtcgctgcc ggtaacaaca acgctgacgc ccgcaactac 780
   tcccctgctt ctgagccctc ggtctgcacc gtcggtgctt ctgaccgcta cgaccgccgc 840
   tccagcttct ccaactacgg cagcgttttg gacatcttcg gccctggtac cagcatcctc 900
   tccacctgga tcggcggcag cacccgctcc atctctggta cctccatggc tactccccac 960
   gttgccggtc tcgctgccta cctcatgact cttggaaaga ctaccgccgc cagcgcttgc 1020
   cgatacattg ccgacaccgc caacaagggc gacttaagca acattccctt cggcactgtc 1080
   aacttgcttg cctacaacaa ctaccaggct taatga 1116
<210> 3
   <211> 83
   <212> DNA
   <213> Artificial Sequence
<220>
   <223> Description of Artificial Sequence: Primer
<400> 3
   atatgaattc atggctcctg ccgttgagca gcgctccgag gctgctcctc tgatcgaggc 60
   ccgcggcgag atggttgcca aca 83
<210> 4
   <211> 80
   <212> DNA
   <213> Artificial Sequence
<220>
   <223> Description of Artificial Sequence: Primer
<400> 4
   atcttctcca tggcagcatc cagagcggaa agagcgctac cctccttgaa cttgacgatg 60
   tacttgttgg caaccatctc 80
<210> 5
   <211> 80
   <212> DNA
   <213> Artificial Sequence
<220>
   <223> Description of Artificial Sequence: Primer
<400> 5
   tgccatggag aagatctctg gcaagcccga ccacgtctac aagaacgtct tcagcggttt 60
   cgctgcgacc ctggacgaga 80
<210> 6
   <211> 64
   <212> DNA
   <213> Artificial Sequence
<220>
   <223> Description of Artificial Sequence: Primer
<400> 6
   tgctcgatgt actcaacatc ggggtgggcg cggagaaccc gaaccatgtt ctcgtccagg 60
   gtcg 64
<210> 7
   <211> 65
   <212> DNA
   <213> Artificial Sequence
<220>
   <223> Description of Artificial Sequence: Primer
<400> 7
   tgagtacatc gagcaggatg ctgttgtcac catcaacgct gcgcagaccg ctgcgcagac 60
   caacg 65
<210> 8
   <211> 70
   <212> DNA
   <213> Artificial Sequence
<220>
   <223> Description of Artificial Sequence: Primer
<400> 8
   agtaggtaga ggtaccgggg ctggtgctgg agatgcgagc caggccccag ggagcgttgg 60
   tctgcgcagc 70
<210> 9
   <211> 80
   <212> DNA
   <213> Artificial Sequence
<220>
   <223> Description of Artificial Sequence: Primer
<400> 9
   gtacctctac ctactactat gacgaatctg ccggccaagg ctcctgcgtc tacgtgatcg 60
   acaccggtat cgaggcatcg 80
<210> 10
   <211> 81
   <212> DNA
   <213> Artificial Sequence
<220>
   <223> Description of Artificial Sequence: Primer
<400> 10
   ttaccgtcgc gactggagta gtagtaggtc ttgaccatct gggcacgacc ctcgaactcg 60
   gggtgcgatg cctcgatacc g 81
<210> 11
   <211> 78
   <212> DNA
   <213> Artificial Sequence
<220>
   <223> Description of Artificial Sequence: Primer
<400> 11
   ccagtcgcga cggtaacggt cacggcaccc actgcgctgg taccgttggc tcccgtacct 60
   acggtgtcgc caagaaga 78
<210> 12
   <211> 73
   <212> DNA
   <213> Artificial Sequence
<220>
   <223> Description of Artificial Sequence: Primer
<400> 12
   atggtggagt actggccact gccgttgtca tccaggacct tgacaccgaa cagctgggtc 60
   ttcttggcga cac 73
<210> 13
   <211> 81
   <212> DNA
   <213> Artificial Sequence
<220>
   <223> Description of Artificial Sequence: Primer
<400> 13
   ggccagtact ccaccatcat cgccggtatg gacttcgttg ccagcgacaa gaacaaccgc 60
   aactgcccca aaggtgtcgt t 81
<210> 14
   <211> 81
   <212> DNA
   <213> Artificial Sequence
<220>
   <223> Description of Artificial Sequence: Primer
<400> 14
   gctctggagg cgggcagcgg cgctgttcac ggaggaggag taaccaccgc ccagggataa 60
   ggaggcaacg acacctttgg g 81
<210> 15
   <211> 82
   <212> DNA
   <213> Artificial Sequence
<220>
   <223> Description of Artificial Sequence: Primer
<400> 15
   gcccgcctcc agagctctgg tgtcatggtc gccgtcgctg ccggtaacaa caacgctgac 60
   gcccgcaact actcccctgc tt 82
<210> 16
   <221> 80
   <212> DNA
   <213> Artificial Sequence
<220>
   <223> Description of Artificial Sequence: Primer
<400> 16
<210> 17
   <211> 83
   <212> DNA
   <213> Artificial Sequence
<220>
   <223> Description of Artificial Sequence: Primer
<400> 17
   ctccagcttc tccaactacg gcagcgtttt ggacatcttc ggccctggta ccagcatcct 60
   ctccacctgg atcggcggca gca 83
<210> 18
   <211> 81
   <212> DNA
   <213> Artificial Sequence
<220>
   <223> Description of Artificial Sequence: Primer
<900> 18
<210> 19
   <211> 81
   <212> DNA
   <213> Artificial Sequence
<220>
   <223> Description of Artificial Sequence: Primer
<400> 19
   ctgcctacct catgacctta ggaaagacca ccgccgccag cgcttgccgt tacatcgccg 60
   acaccgccaa caagggcgac t 81
<210> 20
   <211> 87
   <212> DNA
   <213> Artificial Sequence
<220>
   <223> Description of Artificial Sequence: Primer
<400> 20
   atataagctt ctattaagcc tggtagttgt tgtaggctaa caggttgacg gtgccgaagg 60
   gaatgttgct taagtcgccc ttgttgg 87
<210> 21
   <211> 36
   <212> DNA
   <213> Artificial Sequence
<220>
   <223> Description of Artificial Sequence: Primer
<400> 21
   aattcatgag aggatcgcat cagcatcagc atcagg 36
<210> 22
   <211> 36
   <212> DNA
   <213> Artificial Sequence
<220>
   <223> Description of Artificial Sequence: Primer
<400> 22
   gatccctgat gctgatgctg atgcgatcct ctcatg 36
<210> 23
   <211> 29
   <212> DNA
   <213> Artificial Sequence
<220>
   <223> Description of Artificial Sequence: Primer
<400> 23
   gcggatccgc tcctgccgtt gagcagcgc 29
<210> 24
   <211> 44
   <212> DNA
   <213> Artificial Sequence
<220>
   <223> Description of Artificial Sequence: Primer
<400> 24
   gcggatccga tgacgatgac aaagctcctg ccgttgagca gcgc 44

## Beschreibung

Die vorliegende Erfindung betrifft die Bereitstellung rekombinanter Proteinase K aus *Tritirachium album* Limber sowie entsprechende Verfahren zur Expression, *in vitro* Naturierung und Aktivierung der rekombinanten Proteinase K.

Proteinase K (E.C. 3.4.21.64, bekannt auch als Endopeptidase K) ist eine extrazelluläre Endopeptidase, die von dem Pilz *Tritirachium album* Limber synthetisiert wird. Sie gehört zur Klasse der Serinproteasen mit der typischen katalytischen Triade Asp³⁹-His⁶⁹-Ser²²⁴ (Jany, K.D. et al. (1986) FEBS Letters Vol. 199(2), 139-144). Da die Sequenz der 279 Aminosäuren langen Polypeptidkette (Gunkel, F.A. und Gassen, H.G. (1989) Eur. J. Biochem. Vol. 179(1),185-194) und die dreidimensionale Struktur (Betzel, C. et al. (1988) Eur. J. Biochem. Vol. 178(1), 155-71) eine hohe Homologie mit den bakteriellen Subtilisinen aufweist, wird Proteinase K zur Subtilisinfamilie gezählt (Pahler, A. et al. (1984) EMBO J. Vol. 3(6), 1311-1314; Jany, K.D. und Mayer, B. (1985). Biol. Chem. Hoppe-Seyler, Vol. 366(5), 485-492). Benannt wurde die Proteinase K auf Grund ihrer Fähigkeit, natives Keratin zu hydrolysieren und somit dem Pilz ein Wachstum auf Keratin als einzige C- und N-Quelle zu ermöglichen (Ebeling, W. et al. (1974) Eur. J. Biochem. Vol. 47(1), 91-97, Roelcke und Uhlenbruck, 1969, Z.Med. Mikrobiol.Immunol.Vol.155(2),156-170). Proteinase K ist mit einer spezifischen Aktivität von über 30 U/mg eine der aktivsten bekannten Endopeptidasen (Betzel, C. et al. (1986). FEBS Lett. Vol. 197(1-2), 105- 110) und hydrolysiert unspezifisch native wie denaturierte Proteine (Kraus, E. und Femfert, U, (1976)Hoppe Seylers Z. Physiol.Chem.Vol.357(7):937-947).

Die Proteinase K aus *Tritirachium album* Limber wird im natürlichen Wirt als Präproprotein translatiert. 1989 wurde durch Gunkel, F.A. und Gassen, H.G. (1989) Eur. J. Biochem. Vol. 179(1), 185-194 die Sequenz der cDNA des Gens, das für die Proteinase K codiert, entschlüsselt. Demnach setzt sich das Gen für die Präproproteinase K aus 2 Exons zusammen und codiert für eine 15 Aminosäuren lange Signalsequenz, eine 90 Aminosäuren lange Prosequenz und eine 279 Aminosäuren lange reife Proteinase K. Ein 63 bp langes Intron befindet sich im Bereich der Prosequenz. Das Präpeptid wird bei der Translokation in das endoplasmatische Retikulum (ER) abgespalten. Über die daran anschließende Prozessierung zur reifen Proteinase K unter Abspaltung des Propeptids ist heute noch sehr wenig bekannt.

Die reife Proteinase K besteht folglich aus 279 Aminosäuren. Die kompakte Struktur wird durch zwei Disulfidbrücken und zwei gebundene Calciumionen stabilisiert. Dies erklärt, warum Proteinase K im Vergleich zu anderen Subtilisinen eine deutlich höhere Stabilität gegenüber extremen pH-Werten, hohen Temperaturen, chaotropen Substanzen und Detergenzien zeigt (Dolashka, P. et al. (1992) Int. J. Pept. Protein. Res. Vol. 40(5), 465-471). Proteinase K zeichnet sich durch eine hohe Thermostabilität (bis 65°C, Bajorath et al. (1988), Eur. J. Biochem. Vol. 176, 441-447) und einen weiten pH-Bereich (pH 7,5-12,0, Ebeling, W. et al. (1974) Eur. J. Biochem. Vol. 47(1), 91-97) aus. Die Aktivität wird in Anwesenheit von denaturierenden Substanzen wie Harnstoff oder SDS gesteigert (Hilz, H. et al. (1975) J. Biochem. Vol. 56(1), 103-108; Jany, K.D. und Mayer, B. (1985) Biol. Chem. Hoppe-Seyler, Vol. 366(5), 485-492).

Die oben genannten Eigenschaften machen die Proteinase K insbesondere bei solchen biotechnologischen Anwendungen interessant, bei denen ein unspezifischer Proteinabbau erforderlich ist. Besonders zu erwähnen ist hier die Nukleinsäureisolierung (DNA oder RNA) aus Rohextrakt und die Probenvorbereitung bei der DNA-Analytik (Goldenberger, D. et al. (1995) PCR Methods Appl. Vol. 4(6), 368-370; US 5,187,083; US 5,346,999). Weitere Anwendungen liegen im Bereich der Proteinanalytik wie z.B. der Strukturaufklärung.

Proteinase K wird kommerziell in großen Mengen durch Fermentation des Pilzes *Tritirachium album* Limber (z.B. CBS 348.55, Merck Stamm No. 2429 oder Stamm ATCC 22563) gewonnen. Die Produktion der Proteinase K wird durch Glucose oder freie Aminosäuren supprimiert. Da proteinhaltige Medien die Expression der Proteasen induzieren, müssen als einzige Stickstoffquelle Proteine wie BSA, Milchpulver oder Sojabohnenmehl verwendet werden. Die Sekretion der Protease beginnt, sobald die stationäre Phase des Wachstums erreicht ist (Ebeling, W. et al. (1974) Eur. J. Biochem. Vol. 47(1), 91-97).

Da *Tritirachium album* Limber für eine Fermentation in großem Maßstab schlecht geeignet ist und außerdem genetisch schwer manipulierbar ist, wurden verschiedene Versuche unternommen, eine Überexpression von rekombinante Proteinase K in anderen Wirtszellen zu erreichen. Wegen mangelnder Expression, Bildung von inaktiven "inclusion bodies" oder Problemen bei der Naturierung konnten jedoch bei diesen Versuchen keine signifikante Aktivität nachgewiesen werden (Gunkel, F.A. und Gassen, H.G. (1989) Eur. J. Biochem. Vol. 179(1), 185-194; Samal, B.B. et al. (1996) Adv. Exp. Med. Biol. Vol. 379, 95-104).

Tritirachium album Limber ist zudem ein langsam wachsender Pilz, der nur geringe Mengen an Proteasen in das Medium sezerniert. Nachteilig ist dabei die lange Fermentationszeit von ein bis zwei Wochen. Außerdem ist es bekannt, daß T. album außer Proteinase K auch andere Proteasen produziert, die die Präparation verunreinigen könnten (Samal, B.B. et al. (1991). Enzyme Microb. Technol. Vol. 13, 66-70).

Aufgabe der vorliegenden Erfindung ist es, ein Verfahren zur wirtschaftlichen Produktion von rekombinanter Proteinase K sowie autokatalytisch aktivierbaren inaktiven zymogenen Vorstufen der Proteinase K zur Verfügung zu stellen.

Die Aufgabe wurde gelöst durch die Bereitstellung eines Verfahrens zur Herstellung von rekombinanter Proteinase K, bei dem die inaktive zymogene Proform der Proteinase K in unlöslicher Form in Einschlußkörpern (inclusion bodies) hergestellt wird und wobei dann in anschließenden Schritten die zymogene Proform der Proteinase K naturiert und die zymogene Proform prozessiert d.h. aktiviert wird. Die Verfahren zur Naturierung und Aktivierung der Proteinase K sind ebenfalls Gegenstand der vorliegenden Erfindung. Gegenstand der vorliegenden Erfindung ist ein Verfahren zur Herstellung reombinanter Proteinase K, dadurch gekennzeichnet, daß die zymogene Proform durch in vitro Naturierung gefaltet und durch autokatalytische Spaltung in die aktive Form überführt wird. Gegenstand der vorliegenden Erfindung ist insbesondere ein Verfahren zur Herstellung einer rekombinanten Proteinase K, wobei eine zymogene Vorstufe der Proteinase K aus isolierten und solubilisierten inclusion bodies durch oxydative Faltung in die native Struktur überführt, d.h. naturiert, wird und anschließend durch Zugabe von Detergenzien die aktive Proteinase K aus dem nativ gefalteten Zymogen durch autokatalytische Spaltung erhalten wird.

Gegenstand der vorliegenden Erfindung ist somit ein Verfahren zur Gewinnung von rekombinanter Proteinase K durch Transformation einer Wirtszelle mit einer für die zymogene Proform der Proteinase K codierenden DNA, gekennzeichnet durch folgende Verfahrensschritte:
a) Kultivierung der genannten Wirtszelle unter Bedingungen, die eine Expression der r für die zymogene Proform der Proteinase K codierenden DNA bewirkt, so daß eine zymogene Vorstufe der Proteinase K in der Wirtszelle in Form von unlöslichen inclusion bodies entsteht.
b) Isolierung der inclusion bodies, Solubilisierung des Enzyms und Naturierung r der zymogenen Vorstufe der Proteinase K unter Bedingungen, bei denen der Proteaseanteil der zymogenen Vorstufe der Proteinase K entsteht.
c) Aktivierung der Proteinase K durch Entfernung des Propeptids und weitere Aufreinigung.

Die für die zymogene Proform der Proteinase K codierende DNA entspricht der in SEQ. ID. NO.: 2 dargestellten DNA oder einer im Rahmen der Degeneration des genetischen Codes entsprechenden DNA. SEQ. ID. NO.: 2 umfaßt sowohl die DNA Sequenz, die für die Proteinase K kodiert als auch das Propeptid. Die DNA kann des weiteren für die Expression in einem bestimmten Wirt Codon-optimiert werden. Methoden zur Codon-Optimierung sind dem Fachmann bekannt und in Beispiel 1 beschrieben. Gegenstand der vorliegenden Erfindung sind somit Verfahren, bei denen die Wirtszelle durch eine DNA transformiert wird, welche aus der vorstehend genannten Gruppe ausgewählt ist.

Durch das erfindungsgemäße Verfahren gelingt es, Proteinase K zu erhalten, welche homogen ist und daher in besonderem Maße für analytische und diagnostische Anwendungen geeignet ist. Die erfindungsgemäße zymogene Proform der Proteinase K kann gegebenenfalls weitere N-terminale Modifikationen enthalten, insbesondere Sequenzen, die eine Reinigung des Zielproteins erleichtern ("affinity tags"), Sequenzen, die die Effizienz der Translation erhöhen, Sequenzen, die die Faltungseffizienz steigern oder Sequenzen, die zu einer Sekretion des Zielproteins in das Kulturmedium führen (natürliche Präsequenz und andere Signalpeptide).

Unter Proteinase K im Sinne der Erfindung sind sowohl die in SEQ. ID. NO.: 1 angegebene Sequenz nach Gassen *et al*. (1989), als auch andere Varianten der Proteinase K aus *Tritirachium album* Limber, wie die Ch. Betzel et al. (Biochemistry 40 (2001), 3080-3088) offenbarte Aminosäuresequenz und mit rekombinanten Mitteln erzeugte Varianten (wie zum Beispiel beschrieben in der WO 96/28556) zu verstehen. Die in SEQ. ID. NO.: 1 angegebene Sequenz umfaßt die Signalsequenz (1-15, 15 Aminosäuren), die Prosequenz (16-105; 90 Aminosäuren) und die Sequenz der reifen Proteinase K (106-384, 279 Aminosäuren). Die von Betzel et al. (Biochemistry 40 (2001), 3080-3088) beschriebene Aminosäuresequenz weist insbesondere in Position 207 der aktiven Protease Aspartat anstatt eines Serinrestes auf.

Unter pro-Proteinase K im Sinne der Erfindung ist eine Proteinase K zu verstehen, die am N-Terminus mit ihrer Prosequenz verknüpft ist. Es ist bekannt, daß die Prosequenz bei dem eng verwandten Subtilisin E und anderen Varianten einen essentiellen Einfluß auf die Faltung und Bildung von aktiver Protease hat (Ikemura, H. et al. (1987) Biol. Chem. Vol. 262(16), 7859-7864). Es wird vermutet, daß die Prosequenz als intramolekulares Chaperon wirkt (Inouye, M. (1991) Enzyme Vol. 45, 314-321). Nach der Faltung findet die Prozessierung zur reifen Subtilisin-Protease statt, indem das Propeptid autokatalytisch abgespalten wird (Ikemura, H. und Inouye, M. (1988) J. Biol. Chem. Vol. 263(26), 12959-12963). Dieser Prozess findet bei Subtilisin E (Samal, B.B. et al. (1989) Gene Vol. 85(2), 329-333; Volkov, A. und Jordan, F. (1996) J. Mol. Biol. Vol. 262, 595-599), Subtilisin BPN' (Eder, J. et al. (1993) Biochemistry Vol. 32, 18-26), Papain (Vernet, T. et al. (1991) J. Biol. Chem. Vol. 266(32), 21451-21457) und Thermolysin (Marie-Claire, C. (1998) J. Biol. Chem. Vol. 273(10), 5697-5701) intramolekular statt.

Bei exogener Zugabe kann das Propeptid aber auch intermolekular *in trans* als Chaperon auf die Faltung von denaturierter reifer Subtilisin-Protease wirken (Ohta, Y. et al. (1991) Mol. Microbiol. Vol. 5(6), 1507-1510; Hu, Z. et al. (1996) J. Biol. Chem. Vol. 271(7), 3375-3384). Das Propeptid bindet am aktiven Zentrum von Subtilisin (Jain, S.C. et al. (1998) J. Mol. Biol. Vol. 284, 137-144) und wirkt als spezifischer Inhibitor (Kojima, S. et al. (1998) J. Mol. Biol. Vol. 277,1007-1013; Li, Y. et al. (1995) J. Biol. Chem. Vol. 270, 25127-25132; Ohta, Y. (1991) Mol. Microbiol. Vol. 5(6), 1507-1510). Dieser Effekt wird im Sinne der Erfindung genutzt, um während der Naturierung eine Autoproteolyse von proteolysesensitiven Faltungsintermediaten durch bereits gefaltete, aktive Proteinase K zu verhindern.

Für die Funktion als Chaperon scheinen nur bestimmte, meist hydrophobe Kernbereiche der Prosequenz notwendig zu sein, da Mutationen in weiten Bereichen ohne Einfluß auf die Aktivität bleiben (Kobayashi, T. und Inouye, M. (1992) J. Mol. Biol. Vol. 226, 931-933). Außerdem ist bekannt, daß Propeptide zwischen verschiedenen Subtilisinvarianten austauschbar sind. So erkennt zum Beispiel Subtilisin BPN' auch die Prosequenz von Subtilisin E (Hu, Z. et al. (1996) J. Biol. Chem. Vol. 271(7), 3375-3384). "Inclusion bodies" sind mikroskopisch sichtbare Partikel aus unlöslichen und inaktiven Proteinaggregaten, die oft bei der Überexpression heterologer Proteine zumeist im Cytoplasma der Wirtszelle entstehen und das Zielprotein in hoher Reinheit enthalten. Verfahren zur Herstellung und Reinigung solcher "inclusion bodies" sind beispielsweise beschrieben in Creighton, T.E. (1978) Prog. Biophys. Mol. Biol. Vol. 33(3), 231-297; Marston, FA. (1986) Biochem. J. Vol. 240(1), 1-12; Rudolph, R. (1997). Folding Proteins. In: Creighton, T.E. (ed.), Protein Function: A practical Approach. Oxford University Press,. 57-99; Fink, A.L. (1998) Fold. Des. Vol. 3(1), R9-23; und EP 0 114 506.

Zur Isolierung der "inclusion bodies" werden die Wirtszellen nach der Fermentation durch übliche Methoden, z.B. mittels Ultraschall, Hochdruckdispersion oder Lysozym, aufgeschlossen. Der Aufschluß findet vorzugsweise in einem wässrigen, neutralen bis leicht sauren Puffer statt. Die unlöslichen "inclusion bodies" können nach verschiedenen Methoden abgetrennt und gereinigt werden, vorzugsweise durch Abzentrifugieren oder Filtrieren mit mehreren Waschschritten (Rudolph, R. (1997). Folding Proteins. In: Creighton, T.E. (ed.), Protein Function: A practical Approach. Oxford University Press, 57-99).

Die auf diese Weise gewonnenen "inclusion bodies" werden dann in ebenfalls an sich bekannter Weise solubilisiert. Dazu werden zweckmäßig Denaturierungsmittel in einer Konzentration eingesetzt, die geeignet ist, die "inclusion bodies" aufzulösen, insbesondere Guanidinium-Hydrochlorid und andere Guanidiniumsalze und/oder Harnstoff. Zur vollständigen Monomerisierung der "inclusion body"-Proteine ist es weiterhin vorteilhaft, während der Solubilisierung ein Reduktionsmittel wie Dithiothreitol (DTT), Dithioerythritol (DTE) oder 2-Mercaptoethanol zuzugeben, um eventuell bestehende Disulfidbrücken durch Reduktion aufzubrechen. Ebenfalls Gegenstand dieser Erfindung ist eine Proteinase K, bei der die Cysteine nicht reduziert, sondern derivatisiert sind, insbesondere zu gemischten Disulfiden mit GSSG oder zu Thiocyanaten (EP 0 393 725).

Die Solubilisierung der inclusion bodies erfolgt daher erfindungsgemäß bevorzugt durch denaturierende Agenzien und reduzierende Agenzien. Als denaturierende Agenzien werden 6-8 M Guanidinium Hydrochlorid oder 8-10 M Harnstoff bevorzugt und als reduzierende Agenzien 50-200 mM DTT (Dithiothreitol), oder DTE (Dithioerythritol).

Die vorliegende Erfindung umfaßt somit sowohl die 90 Aminosäuren lange Prosequenz nach SEQ. ID. NO.: 1 (Aminosäuren 16-105), als auch andere Varianten, die eine faltungsfördernde Funktion erfüllen. Ebenfalls umfaßt ist ein Propeptid, das exogen zu der Faltung von reifer Proteinase K gegeben wird und die oben ausgeführten Funktionen erfüllt.

Ein weiterer Gegenstand der Erfindung ist ein rekombinanter Vektor, der eine oder mehrere Kopien der vorstehend definierten rekombinanten DNA enthält. Der Basisvektor ist zweckmäßig ein Plasmid, vorzugsweise mit einem multi-copy-Replikationsursprung, jedoch können auch virale Vektoren eingesetzt werden. Die Auswahl des Expressionsvektors richtet sich nach der ausgewählten Wirtszelle. Zur Herstellung des Expressionsvektors und zur Transformation der Wirtszelle mit diesem Vektor werden Methoden verwendet, die dem Fachmann geläufig sind und zum Beispiel bei Sambrook et al. (1989), Molecular Cloning, s.u. beschrieben sind. Ein geeigneter Vektor für die Expression in *E. coli* ist beispielsweise der pKKT5 Expressionsvektor, oder auch pKK177, pKK223, pUC, pET-Vektoren (Novagen) sowie pQE-Vektoren (Qiagen). Der Expressionsplasmid pKKT5 ist aus pKK177-3 (Kopetzki et al., 1989, Mol. Gen. Genet. 216:149-155) durch Austausch des *tac*-Promotors durch den T5-Promotor aus pDS entstanden(Bujard et al. 1987, Methods Enzymol. 155: 416-433). Die *Eco*RI-Restriktionsendonukleaseschnittstelle in der Sequenz des T5-Promotors wurde durch zwei Punktmutationen entfernt.

Ferner befindet sich die kodierende DNA im erfindungsgemäßen Vektor unter der Kontrolle eines vorzugsweise starken, regulierbaren Promotors. Bevorzugt ist ein IPTG induzierbarer Promotor wie der lac-, lacUV5-, tac- oder T5-Promoter. Insbesondere bevorzugt ist der T5-Promotor.

Als Wirtszelle im Sinne der Erfindung ist jede Wirtszelle zu verstehen, in der Proteine als inclusion bodies entstehen können. Üblicherweise handelt es sich dabei um einen Mikroorganismus, z.B. Prokaryonten. Bevorzugt werden prokaryontische Zellen, insbesondere *Escherichia coli*. Insbesondere werden die folgenden Stämme bevorzugt: *E. coli* K12-Stämme JM83, JM105, UT5600, RR1Δ15, DH5α, C600, TG1, NM522, M15 oder die *E. coli* B-Derivate BL21, HB101, besonders bevorzugt ist *E. coli* M15.

Die entsprechenden Wirtszellen werden erfindungsgemäß transformiert mit einer rekombinanten Nukleinsäure codierend eine rekombinante zymogene Proteinase K gemäß SEQ. ID. NO.: 2 beziehungsweise mit einer Nukleinsäure, welche nach Codonoptimierung aus der vorstehend genannten DNA entstanden ist beziehungsweise mit einer DNA, die im Rahmen der Degeneration des genetischen Codes aus der vorstehend genannten DNA entstanden ist. Die E. coli Wirtszellen werden bevorzugt mit einer codonoptimierten rekombinanten Nukleinsäure codierend eine rekombinante zymogene Proteinase K transformiert, welche optimiert wurde für die Expression in Escherichia coli. Gegenstand der vorliegenden Erfindung ist somit auch ein geeigneter Vektor, beispielsweise ausgewählt aus den oben genannten, der eine für E. coli codonoptimierte rekombinante Nukleinsäure enthält, welche eine rekombinante Proteinase K oder eine rekombinante zymogene Proteinase K codiert. Gegenstand der vorliegenden Erfindung ist des weiteren eine Wirtszelle, beispielsweise ausgewählt aus den oben genannten, der durch den vorstehend genannten Vektor transformiert wurde.

Gegenstand der vorliegenden Erfindung ist des weiteren ein Verfahren zur Naturierung denaturierter zymogener Proteinase K wobei die denaturierte, zymogene Proteinase K in einen Faltungspuffer überführt wird, dadurch gekennzeichnet daß der Faltungspuffer folgende Merkmale aufweist:
- A) pH-Wert des Puffers liegt im Bereich von 7,5 und 10,5,
- B) Anwesenheit von niedermolekularen Faltungshilfsstoffen,
- C) Anwesenheit eines Redox-Shuffling-Systems
- D)Anwesenheit eines Komplexbildners in einer substöchiometrischen Konzentration zu anwesenden Ca²⁺-Ionen
- E) Anwesenheit von SDS in einer Konzentration zwischen 0,1% und 2% [w/v], und wobei das Verfahren bei einer Temperatur zwischen 0°C und 37°C durchgeführt wird.

Bevorzugt liegt während der Naturierung eine niedrige Konzentration von Denaturierungsmitteln vor. Denaturierungsmittel können beispielsweise deshalb zugegen sein, weil sie sich aufgrund der vorhergehenden Solubilisierung der inclusion bodies noch in der Reaktionslösung befinden. Die Konzentration an Denaturierungsmitteln wie beispielsweise Guanidinhydrochlorid sollte kleiner als 50 mM sein.

Unter Naturierung im Sinne der Erfindung wird ein Verfahren verstanden, bei welchem denaturiertes, im wesentlichen inaktives Protein in eine Konformation überführt wird, in der das Protein nach autokatalytischer Spaltung und Aktivierung die gewünschte Aktivität zeigt. Dazu werden die solubilisierten inclusion bodies unter Verringerung der Konzentration des Denaturierungsmittels in einen Faltungspuffer überführt. Die Bedingungen müssen so gewählt sein, daß das Protein dabei in Lösung bleibt. Zweckmäßig kann dies durch eine schnelle Verdünnung oder durch Dialyse gegen den Faltungspuffer erfolgen.

Bevorzugt ist, daß der Faltungspuffer einen pH- Wert von pH 8 bis pH 9 aufweist. Insbesondere bevorzugt als Puffersubstanzen sind Tris/HCI-Puffer und Bicin-Puffer.

Bevorzugt wird das erfindungsgemäße Naturierungsverfahren bei einer Temperatur zwischen 0°C und 25°C durchgeführt.

Die im Faltungspuffer enthaltenen niedermolekularen Faltungshilfsstoffe werden bevorzugterweise ausgewählt aus der folgenden Gruppe niedermolekularer Verbindungen und können sowohl allein als auch in Mischungen zugesetzt werden, wobei noch weitere Faltungshilfsstoffe zugegen sein können:
- L-Arginin in einer Konzentration von 0,5 M bis 2,0 M
- Tris in einer Konzentration von 0,5 M bis 2,0 M
- Triethanolamin in einer Konzentration von 0,5 M bis 2,0M
- α-Cyclodextrin einer Konzentration von 60 mM bis 120 mM.

Niedermolekularen Faltungsshilfsstoffe werden zum Beispiel in US 5,593,865; Rudolph, R. (1997) Folding Proteins. In: Creighton, T.E. (ed.), Protein Function: A practical Approach. Oxford University Press, 57-99 oder De Bernardez Clark, E. et al. (1999) Methods. Enzymol. Vol. 309, 217-236 beschrieben.

Bevorzugt handelt es sich bei dem oben genannten Redox-Shuffling-System um gemischte Disulfide oder Thiosulfonate.

Als Redox-Shuffling-System eignen sich beispielsweise Systeme, bestehend aus einer Thiolkomponente in oxidierter und reduzierter Form. Dadurch wird einerseits durch die Kontrolle des Reduktionspotentials die Ausbildung von Disulfidbrücken innerhalb der sich faltenden Polypeptidkette während der Naturierung erlaubt, und andererseits das "reshuffling" inkorrekter Disulfidbrücken innerhalb oder zwischen den sich faltenden Polypeptidketten ermöglicht (Rudolph, R. (1997), s.o.). Bevorzugte Thiolkomponenten sind zum Beispiel:
- Glutathion in reduzierter (GSH) und oxidierter Form (GSSG)
- Cystein und Cystin
- Cysteamin und Cystamin
- 2-Mercaptoethanol und 2-Hydroxyethyldisulfid.

Bei dem erfindungsgemäßen Naturierungsverfahren liegen die Ca²⁺-Ionen bevorzugterweise in einer Konzentration von 1 bis 20 mM vor. Beispielsweise kann CaCl₂ in Mengen von 1 bis 20 mM zugegeben werden. Die Ca²⁺ Ionen können an die Calciumbindungsstellen der sich faltenden Proteinase K binden.

Die Gegenwart eines Komplexbilders, vorzugsweise EDTA in einer substöchiometrischen Konzentration zu Ca²⁺, dient der Verhinderung der Oxidation des Reduktionsmittels durch Luftsauerstoff und dem Schutz freier SH-Gruppen.

Die Naturierung wird bevorzugt bei niedriger Temperatur, d.h. unterhalb von 20°C, vorzugsweise 10°C bis 20°C durchgeführt. Bei dem erfindungsgemäßen Verfahren ist die Naturierung in der Regel nach einer Dauer von etwa 24 h bis 48 h beendet.

Gegenstand der vorliegenden Erfindung ist weiterhin der Faltungspuffer, der durch folgende Merkmale gekennzeichnet ist:
- A) pH-Wert des Puffers liegt im Bereich von 7,5 und 10.5,
- B) Anwesenheit von niedermolekularen Faltungshilfsstoffen,
- C) Anwesenheit eines Redox-Shuffling-Systems
- D)Anwesenheit eines Komplexbildners in einer substöchiometrischen Konzentration zu anwesenden Ca2+-Ionen,
- E) Anwesenheit von SDS in einer Konzentration zwischen 0,1% und 2% [w/v],
- F) Proteinase K.

Insbesondere ist bevorzugt, wenn der Faltungspuffer einen pH- Wert von pH 8 bis pH 9 aufweist und/oder wenn es sich bei dem Redox-Shuffling-System um gemischte Disulfide oder Thiosulfonate handelt.

Gegenstand der Erfindung ist weiterhin ein Verfahren zur Aktivierung der naturierten zymogenen Vorstufe der Proteinase K. Nach dem erfindungsgemäßen Faltungsprozess bildet sich ein inaktiver Komplex aus nativer Proteinase K und dem inhibitorisch wirkenden Propeptid. Die aktive Proteinase K kann aus diesem Komplex freigesetzt werdendurch Zugabe von SDS in einer Konzentration von 0, 1 bis 2 % (w/v).

Die Vorteile der hier offen gelegten Verfahren zur Herstellung rekombinanter Proteinase K sind:
1. die Möglichkeit, das hohe Expressionspotential und die schnelle und einfache Kultivierung von *Escherichia coli* oder anderer geeigneter Mikroorganismen zu nutzen.
2. die Möglichkeit der genetischen Manipulation der rekombinanten DNA.
3. der geringe Reinigungsaufwand nach der Naturierung.
4. die Abwesenheit eukaryontischer Verunreinigungen bei einer der Wahl eines Prokaryonten als Wirtszelle.

Denkbar wäre auch ein Verfahren dadurch gekennzeichnet daß die Nukleinsäuren, die einerseits für die reife Proteinase K kodieren und andererseits für das Propeptid der Proproteinase K kodieren getrennt in Wirtszellen exprimieret werden und dann zur Naturierung der reifen Proteinase K gemeinsam in einen Faltungspuffer überführt werden.

### Beschreibung der Figuren:

Figur 1:
   Schematische Darstellung der PCR-Reaktion zur Herstellung von Proteinase K-Fragmenten mit N-terminaler *Bam*HI-Schnittstelle und alternativer Enterokinase-Spaltstelle zur Fusionierung mit einem N-terminalen Affinitäts-tag.
Figur 2:
   Ausbeute der Naturierung in Abhängigkeit von der Temperatur.
Figur 3:
   Ausbeute der Naturierung in Abhängigkeit vom pH-Wert.
Figur 4:
   Ausbeute der Naturierung in Abhängigkeit vom Redoxpotential.
Figur 5:
   Ausbeute der Naturierung in Abhängigkeit von der Argininkonzentration.
Figur 6:
   Ausbeute der Naturierung in Abhängigkeit von der Triskonzentration.
Figur 7:
   Ausbeute der Naturierung in Abhängigkeit von der α-Cyclodextrinkonzentration.
Figur 8:
   Ausbeute der Naturierung in Abhängigkeit von der Triethanolaminkonzentration.
Figur 9:
   Ausbeute der Naturierung in Abhängigkeit von der Ureakonzentration.
Figur 10:
   SDS Polyacralymidgel der Naturierung von pro-Proteinase K.
Figur 11:
   Reversed phase Chromatographie von naturierter Proteinase K.
Figur 12:
   Renaturierte und prozessierte Proteinase K wurde mittels analytischer Ultrazentrifugation analysiert. Die Zentrifugation wurde bei 12 000 rpm, 20°C für 63 h durchgeführt. Die ermittelten Daten (o) konnten zu einer homogenen Spezies mit einem apparenten Molekulargewicht von 29 490 Da gefittet werden. Es ist keine systematische Abweichung der gefitteten von den gemessenen Daten zu beobachten (unterer Graph).
Figur 13:
   Bestimmung des Km-Wertes der naturierten Proteinase K.
Figur 14:
   Abbaumuster von Blutserumproteinen durch naturierte Proteinase K.
Figur 15:
   Reinigung der naturierten Proteinase K durch Gelfiltration.

### Beispiel 1:

### Synthese des Gens, welches für die reife Form der Proteinase K codiert.

Das Gen für die reife Proteinase K aus *Tritirachium album* Limber ohne Signalsequenz und ohne Intron wurde mittels Gensynthese generiert. Als template wurde die 837 bp lange Sequenz von Gunkel, F.A. und Gassen, H.G. (1989) Eur. J. Biochem. Vol. 179(1), 185-194 verwendet (Aminosäuren 106-384 aus Swiss Prot P06873). Zur Optimierung der Expression wurde bei der Rücktranslation eine für *Escherichia coli* optimierte codon usage zugrunde gelegt (Andersson, S.G.E. und Kurland, C.G. (1990) Microbiol. Rev. Vol. 54(2),198-210, Kane, J.F.Curr. Opin. Biotechnol., Vol. 6, pp. 494-500). Die Aminosäuresequenz ist in SEQ. ID. NO.: 1, die Nukleotidsequenz in SEQ. ID. NO.: 2 dargestellt.

Für die Gensynthese wurde das Gen in 18 Fragmente aus Sinn- und reversen, komplementären Gegenstrangoligonukleotiden in alternierender Folge unterteilt (SEQ. ID. NO.: 3-20). Am 5'- und 3'-Ende wurde jeweils ein mindestens 15 bp- langer Bereich angehängt, der jeweils mit den benachbarten Oligonukleotiden überlappt. An die 5'- und 3'-Enden des synthetischen Gens wurden außerhalb des codierenden Bereichs zur späteren Klonierung in Expressionsvektoren Erkennungsstellen für Restriktionsendonukleasen angehängt. Für die Klonierung des pro-Protein X-Gens ohne N-terminalen Affinitäts-tag wurde als 5'-Primer das in SEQ. ID. NO.: 3 dargestellte Oligonukleotid verwendet, das eine *Eco*RI-Schnittstelle enthält. SEQ. ID. NO.: 20 zeigt den 3'-Primer mit *Hin*dIII-Schnittstelle. Der 3'-Primer enthält ein zusätzliches Stoppcodon nach dem natürlichen Stoppcodon, um eine gesicherte Termination der Translation zu gewährleisten. Für die Klonierung des pro-Protein X-Gens mit N-terminalen Affinitätstags und alternativer Enterokinase-Spaltstelle, die in Beispiel 3 beschrieben ist, wurde als 5'-Primer das in SEQ. ID. NO.: 23 dargstellte Oligonukleotid mit *Bam*HI-Schnittstelle oder das in SEQ. ID. NO.: 24 beschriebene Oligonukleotid mit *Bam*HI-Schnittstelle und Enterokinase-Spaltstelle verwendet.

Die Oligonukleotide wurden mittels PCR-Reaktion miteinander verknüpft und das daraus resultierende Gen amplifiziert. Dabei wurde das Gen zunächst in drei Fragmente zu je 6 Oligonukleotiden unterteilt und in einem zweiten PCR-Zyklus die drei Teilstücke miteinander verknüpft. Fragment 1 setzt sich aus den Oligonukleotiden wie in SEQ. ID. NO.: 3-8 dargestellt, Fragment 2 aus Oligonukleotide wie in SEQ. ID. NO.: 9-14 dargestellt und Fragment drei aus Oligonukleotid wie in SEQ. ID. NO.: 15-20 dargestellt, zusammen.

### Folgende PCR-Parameter wurden angewandt:

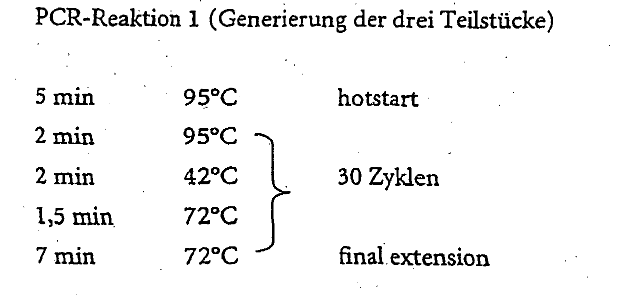

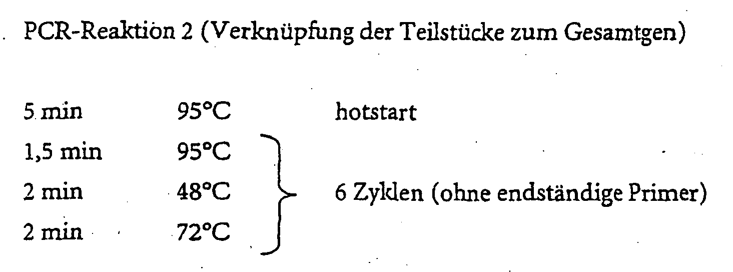

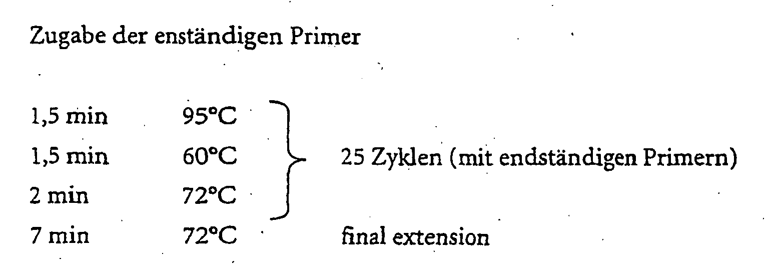

### Beispiel 2:

### Klonierung des synthetischen Proteinase K-Fragmentes aus der Gensynthese

Der PCR-Ansatz wurden auf ein Agarosegel aufgetragen und das ca. 1130 Bp große PCR-Fragment wurde aus dem Agarosegel (Geneclean II Kit von Bio 101, Inc. CA USA) isoliert Das Fragment wurde mit den *Eco*RI- und *Hin*dIII-Restriktionsendonukleasen (Roche Diagnostics GmbH, Germany) 1 Stunde bei 37°C geschnitten. Gleichzeitig wurde das pUC18-Plasmid (Roche Diagnostics GmbH, Germany) mit den *Eco*RI- und *Hin*dIII-Restriktionsendonukleasen 1 Stunde bei 37°C geschnitten, der Ansatz mittels Agarosegelelektorphorese aufgetrennt und das 2635 Bp große Vektorfragment isoliert. Anschließend wurden das PCR-Fragment und das Vektorfragment mittels T4-DNA-Ligase miteinander ligiert. Dazu wurden 1 µl (20 ng) Vektorfragment und 3 µl (100 ng) PCR-Fragment,1 µl 10 x Ligase-Puffer (Maniatis, T., Fritsch, E. F. and Sambrook, T. (1989). Molecular Cloning: A laboratory manual. 2nd ed., Cold Spring Harbor Press, Cold Spring Harbor, N.Y), 1 µl T4-DNA-Ligase, 4 µl steriles H₂O bidest. pipettiert, vorsichtig gemischt und über Nacht bei 16°C inkubiert

Das klonierte Gen wurde mittels Restriktionsanalyse und durch mehrfache Sequenzierung beider Stränge untersucht. Die Sequenz ist in SEQ. ID. NO.: 2 dargestellt.

### a) Konstruktion des pPK-1-Expressionsplasmides

Zur Expression der Proteinase K wurde das Strukturgen in den pKKT5 Expressionsvektor so kloniert, daß das Strukturgen in der richtigen Orientierung unter Kontrolle eines geeigneten Promotors, bevorzugt eines IPTG induzierbaren Promotors wie lac-, lacUV5-, tac- oder T5-Promotor, besonders bevorzugt des T5-Promotors insertiert ist. Dafür wurde das Strukturgen für die Proteinase K mittels *Eco*RI und *Hin*dIII aus dem Plasmid pUC18 ausgeschnitten, der Restriktionsansatz durch Agarosegelelektrophorese aufgetrennt und das ca. 1130 Bp große Fragment aus dem Agarosegel isoliert. Gleichzeitig wurde das Expressionsplasmid pKKT5 mit *Eco*RI und *Hin*dIII geschnitten, der Restriktionsansatz durch Agarosegelelektrophorese aufgetrennt und das ca. 2,5 kBp Vektorfragment aus dem Agarosegel isoliert. Die so gewonnenen Fragmente wurden wie beschrieben miteinander ligiert. Die ordnungsgemäße Insertion des Gens wurde mittels Sequenzierung nachgeprüft.

### b) Transformation des Expressionsplasmides pPK-1 in verschiedene E. coli Expressionsstämme

Der Expressionsvektor wurde in verschiedene Expressionsstämme transformiert, die mit dem Plasmid pREP4 und/oder pUBS520 vortransformiert waren. Das Plasmid pREP4 enthält ein Gen für den *lac*I-Repressor, der eine vollständige Unterdrückung der Expression vor der Induktion sicherstellen soll. Das Plasmid pUBS520 (Brinkmann, U. et al. (1989) Gene Vol. 85(1), 109-114) enthält ebenfalls den *lac*I-Repressor und zusätzlich das dnaY-Gen, das für die tRNA codiert, die für die Translation der in *E*. *coli* seltenen Arginin-codons AGA und AGG notwendig ist. Kompetente Zellen verschiedener *E. coli*-Stämme wurden entsprechend der Methode nach Hanahan, D. (1983) J. Mol. Biol. Vol. 166, 557-580 hergestellt. 100 µl derart hergestellter Zellen wurden mit 20 ng isoliertem pPK-1 Plasmid-DNA versetzt. Nach 30 min Inkubation auf Eis erfolgte ein Hitzeschock (90 sec bei 42°C) gefolgt von 2 min Inkubation auf Eis. Anschließend wurden die Zellen in 1 ml SOC-Medium überführt und zur phänotypischen Expression 1 Stunde bei 37°C unter Schütteln inkubiert. Aliquote dieses Transformationsansatzes wurden auf LB-Platten mit Ampicillin als Selektionsmarker ausplattiert und 15 Stunden bei 37°C inkubiert. Bevorzugte Stämme sind *E. coli* K12-Stämme JM83, JM105, UT5600, RR1Δ15, DH5α, C600, TG1, NM522, M15 oder die *E. coli* B-Derivate BL21, HB101, besonders bevorzugt *E. coli* M15.

### Beispiel 3:

### Klonierung eines N-terminalen Affinitäts-tags.

Zur Einfügung eines N-terminalen Affinitäts-tags wurde vor dem 5'-Ende des Gens für pro-Proteinase K eine *Bam*HI-Schnittstelle eingefügt. Dies wurde mittels PCR mit dem in Beispiel 1 erhaltene Produkt als Template und den in SEQ. ID. NO.: 20, 23 und 24 beschriebenen Oligonukleotiden als Primer erreicht. Der in SEQ. ID. NO.: 23 beschriebene Primer enthält eine *Bam*HI-Schnittstelle vor dem 5'-Bereich der pro-Proteinase K, der in SEQ. ID. NO.: 24 beschriebene Primer zusätzlich eine Enterokinasespaltstelle direkt vor dem ersten Codon der Prosequenz. SEQ. ID. NO.: 20 zeigt den in ebenfalls Beispiel 1 verwendeten 3'-Primer mit *Hin*dIII-Schnittstelle. Die erhaltenen PCR-Produkte wurden wie oben beschrieben isoliert, mit *Bam*HI und *Hin*dIII verdaut und durch Agarose Elektrophorese gereinigt

Der Affinitäts-tag wurde durch einen synthetischen Linker eingefügt, der aus zwei komplementären Oligonukleotiden in der Art zusammengesetzt wurde, daß ohne weiteren Restriktiönsverdau am 5'-Ende eine *Eco*RI-Schnittstelle und am 3'-Ende eine *Bam*HI-Schnittstelle entstand. Für einen His-tag hatte der Sinnstrang die in SEQ. ID. NO.: 21, der Antisinnstrang die in SEQ. ID. NO.: 22 angegebene Sequenz. Der Linker codierte für einen Hexa-Histag mit N-terminalem RGS-Motiv. Die *Bam*HI-Schnittstelle zwischen Linker und pro-Proteinase K wird zu einem Gly-Ser-Linker translatiert. Zum Annealing des Linkers wurden die beiden Oligonukleotide (SEQ. ID. NO.: 21 und 22) in äquimolaren Mengen (je 50 pmol/µl) für 5 min auf 95°C erhitzt und anschließend mit 2°C pro min auf Raumtemperatur abgekühlt Dadurch sollte ein möglichst vollständiges Annealing der komplementären Oligonukleotide erreicht werden.

Der Linker wurde mit dem *Bam*HI/*Hin*dIII-verdauten PCR-Produkt ligiert (Rapid Ligation Kit von Roche Diagnostics GmbH, Deutschland) und durch Agarose Gelelektrophorese gereinigt. (QIAquick Gelextraction Kit von Qiagen, Deutschland). Das so erhaltene Ligationsprodukt wurde über die *Eco*RI und *Hin*dIII-Überhänge analog zu Beispiel 2b in einen Expressionsvektor ligiert und entsprechend in Expressionsstämme transformiert.

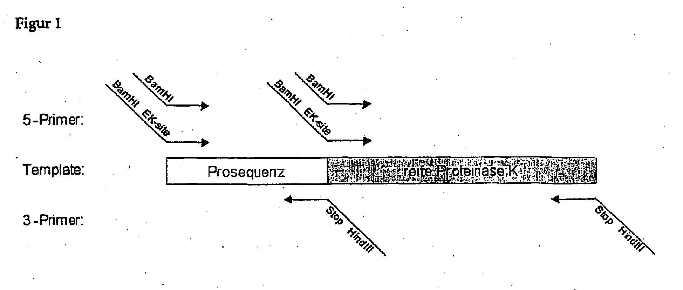

Durch dieses Modulsystem ist es möglich, verschiedene Affinitäts-tags, die von dem synthetischen Linker codiert werden, an das Strukturgen für pro-Proteinase K zu fusionieren. Durch die Wahl des entsprechenden 5'-Primers kann alternativ eine Enterokinasespaltstelle zwischen Tag und Propeptid eingefügt werden, falls ein späteres Entfernen des Tags gewünscht wird. Desweiteren kann durch Wahl der Überlappungsbereiche der PCR-Primer ein bestimmter Bereich des Proteinase K Gens wie zum Beispiel die reife Proteinase K oder das Propeptid amplifiziert werden (Figur 1).

### Beispiel 4:

### Expression von Proteinase K in Escherichia coli

Da die Proteinase K eine sehr aktive unspezifische Protease ist, sollte eine Expression in inaktiver Form bevorzugt als inclusion bodies angestrebt werden.

Zur Expression des Gens, das für die Proteinase K codiert, wurden plasmidhaltige Klone in 3m1 Lbₐₘₚ -Medium angeimpft und bei 37°C im Schüttler inkubiert.
- LB-Medium:: 10 g Trypton
10 g Hefeextrakt
5 g NaCl
ad 11 mit dest. H₂O, pH 7,0 mit NaOH
Zugabe von Antibiotika (100 µg/ml Ampicillin) direkt vor Inokulation

Bei einer optischen Dichte von 0,5 bei 550 nm wurden die Zellen mit 1mM IPTG induziert und 4 h bei 37°C im Schüttler inkubiert. Anschließend wurde die optische Dichte der einzelnen Expressionsklone bestimmt, ein Aliquot, das einer OD₅₅₀ von 3/ml, entspricht, entnommen und die Zellen abzentrifugiert (10 min 6000 rpm, 4°C). Die Zellen wurden in 400 µL TE-puffer resuspendiert, durch Ultraschall aufgeschlossen und durch Zentrifugation die lösliche Proteinfraktion von der unlöslichen Proteinfraktion getrennt (10 min, 14000 rpm, 4°C).
- TE-Puffer: 50 mM Tris/HCl: 50 mM EDTA
pH 8,0 (bei RT)

Alle Fraktionen wurden mit SDS- und β-Mercaptoethanol-haltigem Auftragspuffer versetzt und die Proteine durch Erhitzen (5 min 95°C) denaturiert. Anschließend wurden je 10µl mittels eines 12,5%igen analytisches SDS-Gel analysiert (Laemmli, U.K. (1970) Nature Vol. 227(259), 680-685).

Sowohl für die Klone der reifen Proteinase K als auch für die Klone der pro-Proteinase K konnte eine sehr starke Expression in Form von unlöslichen Proteinaggregaten ("inclusion bodies") beobachtet werden. Dementsprechend wurde auch keine Proteinase K-Aktivität gemessen.

### Beispiel 5:

Isolierung der inclusion bodies

Die Präparation der inclusion bodies wurde nach an sich bekannten Verfahren durchgeführt (Rudolph, R. (1997) s.o.

Für den Zellaufschluß wurden jeweils 10 g Biofeuchtmasse in 50 ml 100 mM Tris/HCl pH 7,0, 1 mM EDTA resuspendiert. Danach wurden 15 mg Lysozym zugegeben, 60 min bei 4°C inkubiert und die Zellen anschließend mittels Hochdruck (Gaulin Zellaufschlußgerät) aufgeschlossen. Die DNA wurde durch Zugabe von 3 mM MgCl₂ und 10 µg/ml DNase zum Rohextrakt 30 min bei RT verdaut. Die unlöslichen Zellbestandteile, die die inclusion bodies enthalten, wurden durch Zentrifugation (30 min 20.000 g) abgetrennt und 1 x mit Waschpuffer 1 und 3 x mit Waschpuffer 2 gewaschen.
- Waschpuffer 1:: 100 mM Tris/HCl
20 mM EDTA
2% (v/v) Triton X-100
0,5 M NaCl
pH 7,0 (RT)
- Waschpuffer 2:: 100 mM Tris/HCl
1 mM EDTA
pH 7,0 (RT)

Das Pellet des letzten Waschschrittes sind die Roh-inclusion bodies, die das Zielprotein bereits in großer Reinheit enthalten.

### Beispiel 6:

Solubilisierung der inclusion bodies

### a) Solubilisierung unter Reduktion der Cysteine

1 g Roh-inclusion bodies wurden in 10 ml Solubilisierungspuffer suspendiert und unter leichtem Rühren 2 h bei RT inkubiert.
- Solubilisierungspuffer:: 100 mM Tris/HCl
6,0 M Guanidiniumhydrochlorid
100 mM DTT
pH 8,0 (RT)

Das Solubilisat wurde mit 25% HCl auf pH 3 titriert und 2 x für 4 h bei RT gegen 500 ml 6 M Guanidinhydrochlorid pH 3 und dann über Nacht bei 4°C gegen 1000 ml Guanidinhydrochlorid pH 7 dialysiert. Die Proteinkonzentration wurde nach der Bradford Methode (Bradford, 1976) und durch einen berechneten Extinktionskoeffizienten bei 280 nm bestimmt und betrug zwischen 10 und 20 mg/ml. Die Anzahl der freien Cysteine wurde nach Ellman bestimmt. Es konnten in Übereinstimmung mit der Sequenz 5 mol freie Cysteine pro mol Proteinase K gefunden werden. Die Reinheit der solubiliserten inclusion bodies wurde durch 12,5% SDS PAGE und Quantifizierung der Banden nach Coomassie-Färbung ermittelt.

### b) Solubilisierung unter Derivatisierung der Cysteine zu gemischten Disulfiden mit Glutathion 1 g Roh-inclusion bodies wurden in 10 ml Solubilisierungspuffer suspendiert.

- Solubilisierungspuffer: 100 mM Tris/HCl: 6,0 M Guanidinhydrochlorid
1 mM DTT
pH 8,0 (RT)

Nach 15 min Inkubation bei RT unter leichtem Rühren, in denen durch die geringen Mengen DTT katalytische Menge reduzierter Cysteine entstanden, wurde 100 mM GSSG zugegeben, der pH auf 8,0 justiert und weitere 2 h unter leichtem Rühren bei RT inkubiert.

Weitere Behandlung wie unter a).

### Beispiel 7:

### Optimierung der Naturierung von pro-Proteinase K

Zur Optimierung der Ausbeute bei der Faltung und Prozessierung der pro-Proteinase K aus den in Beispiel 6 a) hergestellten Solubilisaten wurden verschiedene Parameter variiert. Für alle Ansätze wurde der jeweils angegebene Faltungspuffer filtriert, entgast, mit N₂ begast und bei der gewünschten Temperatur inkubiert. Erst kurz vor Start der Faltungsreation wurde das Redox-shuffling system zugegeben und der pH nachgestellt. Die Faltung wurde durch Zugabe der solubilisierten IB's unter schnellem Mischen gestartet. Das Volumen der Faltungsansätze betrug 1,8 ml in 2 ml Glasröhrchen mit Schraubdeckel. Die Analyse der Ausbeute erfolgte durch einen Aktivitätstest mit dem chromatogenen Substrat Suc-Ala-Ala-Pro-Phe-pNA von der Firma Bachem (Heidelberg). Als Testpuffer wurde 100 mM Tris/HCl, 5 mM CaCl₂, pH 8,5 bei 25°C verwendet.
Die Konzentration des Peptids im Test betrug 2 mM aus einer 200 mM Stammlösung in DMSO. Zum Aktivieren des Renaturats wurde der Probe 0,1% SDS zugegeben (siehe Beispiel 8). Zur Messung wurde die Extinktion bei 410 nm über einen Zeitraum von 20 min verfolgt und aus der Steigung die Aktivität berechnet.

Folgende Parameter wurden variiert:

### a) Temperatur und Zeit

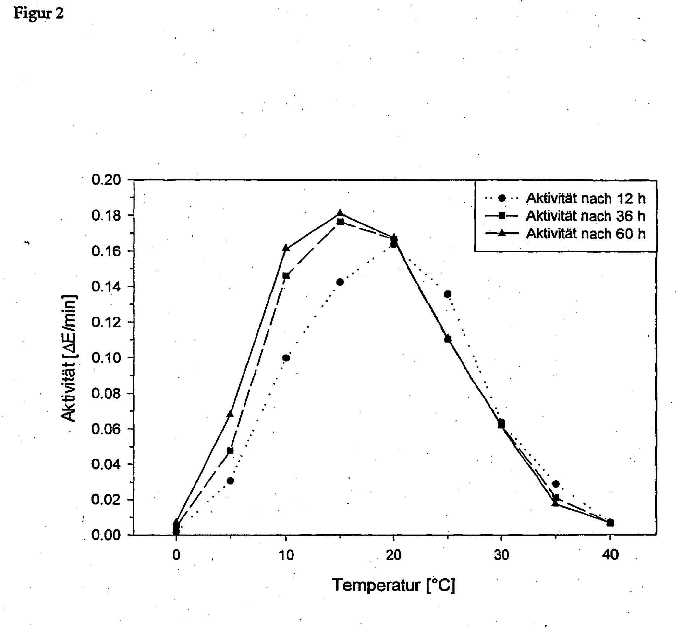

Der Faltungspuffer mit 100 mM Tris, 1,0 mM L-Arginin, 10 mM CaCl₂ wurde bei verschiedenen Temperaturen äquilbriert. Nach Zugabe von 3 mM GSH und 1 mM GSSG wurde der pH bei der entsprechenden Temperatur nachgestellt. Die Reaktion wurde durch Zugabe von 50 µg/ml pro-Proteinase K gestartet. Nach 12 h, 36 h und 60 h wurden Aliquots entnommen und auf Aktivität getestet Die Ergebnisse sind in Figur 2 dargestellt:

### b) pH-Wert

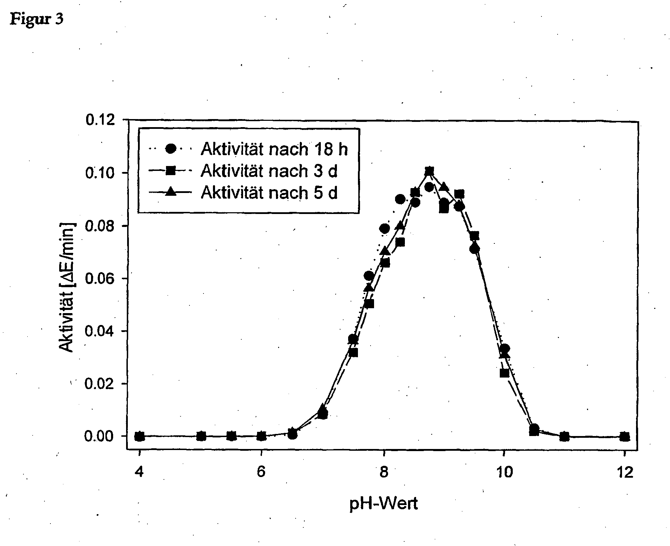

Ein Universalpuffer mit 50 mM Citrat, 50 mM MES, 50 mM Bicin, 500 mM Arginin, 2 mM CaCl₂ und 1 mM EDTA wurde bei 15°C temperiert und mit 3 mM GSH und 1 mM GSSG versetzt. Die pH-Werte im Bereich zwischen pH 4,0 und pH 12,0 wurden nachgestellt die Faltungsreaktion durch Zugabe von 50 µg/ml pro-Proteinase K inclusion bodies gestartet. Die nach 18 h, 3 d und 5 d gemesse Aktivität ist in Figur 3 dargestellt.

### c) Redoxpotential

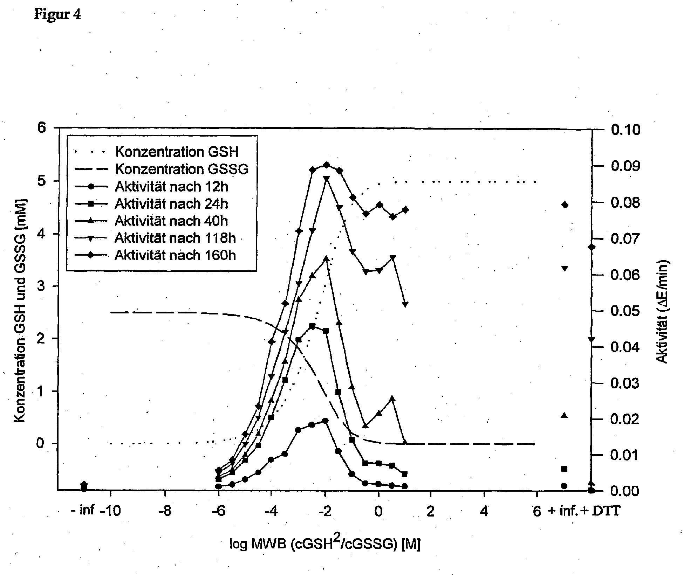

In einem Renaturierungspuffer mit 1,0 M L-Arginin, 100 mM Bicin, 2 mM CaCl₂ und 10 mM CaCl₂ wurden verschiedene Redoxpotentiale durch Mischung verschiedener Verhältnisse von oxidiertem und reduziertem Glutathion eingestellt. Die Proteinkonzentration im Faltungsansatz betrug 50 µg/ml. Die Faltung wurde bei 15°C durchgeführt. Die Konzentrationen an GSH und GSSG sind in Tabelle 1, die Meßwerte in Figur 4 dargestellt.

**Tabelle 1: Konzentrationen von GSH und GSSG bei den verschiedenen Redoxpotentialen**

| Redoxpotential (log(cGSH2/cGSSG) [M]) | c(GSH) [mM] | c(GSSG) [mM] |
|---|---|---|
| - 00 | 0 | 2.500 |
| - 6.000 | 0.050 | 2.475 |
| - 5.500 | 0.088 | 2.456 |
| - 5.000 | 0.156 | 2.422 |
| - 4.500 | 0.273 | 2.363 |
| - 4.000 | 0.476 | 2.262 |
| - 3.500 | 0.814 | 2.093 |
| - 3.000 | 1.351 | 1.825 |
| - 2.500 | 2.130 | 1.435 |
| - 2.000 | 3.090 | 0.955 |
| - 1.500 | 3.992 | 0.504 |
| -1.000 | 4.580 | 0.210 |
| - 0.500 | 4.851 | 0.074 |
| 0.000 | 4.951 | 0.025 |
| + 0.500 | 4.984 | 7.856e-3 |
| + 1.000 | 4.995 | 2.495e-3 |
| + 00 | 5.000 | 0 |

### d) Faltungsfördernde Lösungsmitteladditive

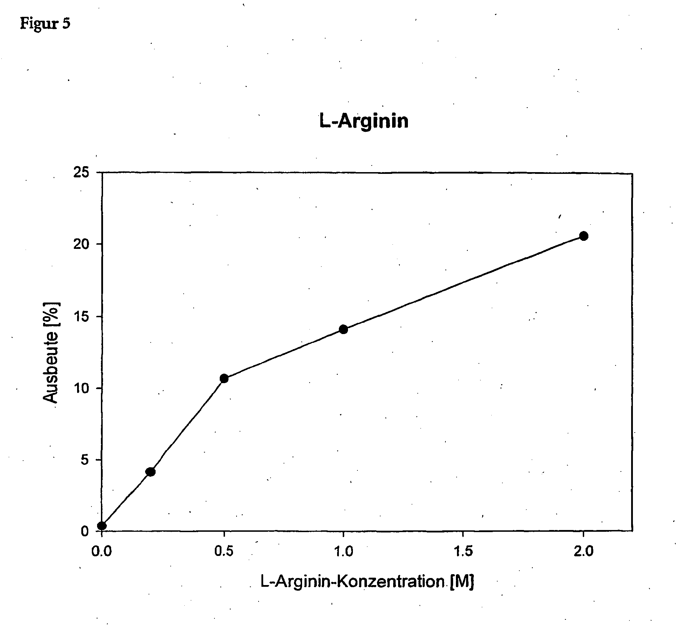

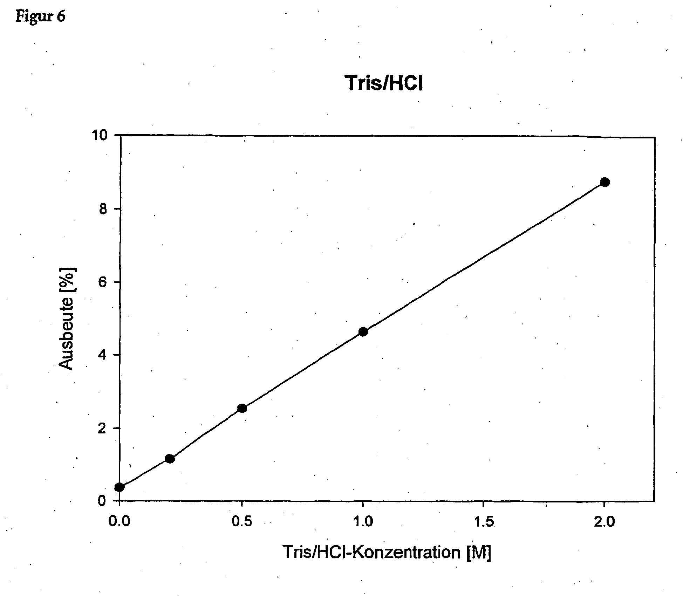

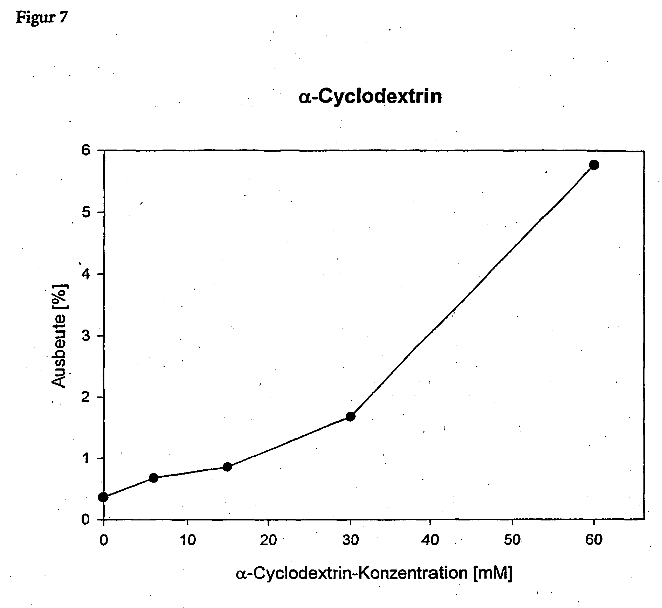

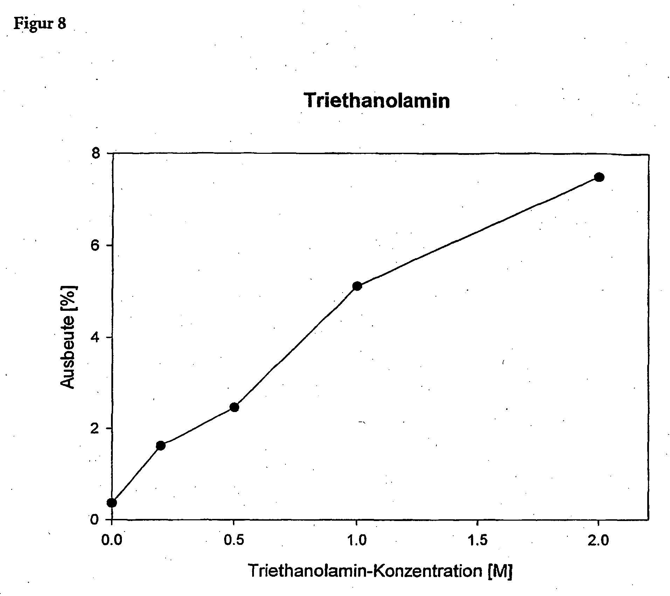

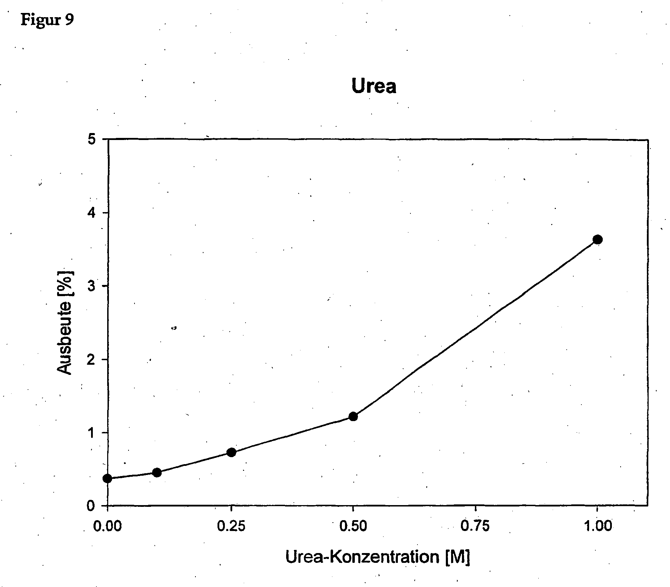

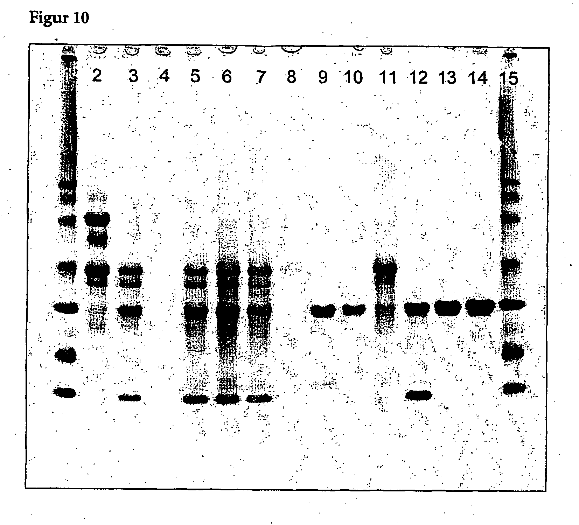

Verschiedene Substanzen wurden auf ihre Fähigkeit überprüft, die Faltungsausbeute von pro-Proteinase K zu steigern. Dazu wurden Lösung dieser Substanzen in verschiedenen Konzentrationen hergestellt und mit 2 mM CaCl₂, 1 mM EDTA und 100 mM Bicin versetzt. D er pH-Wert wurde auf pH 8,75 bei der Faltungstemperatur von 15°C eingestellt. Die Proteinkonzentration betrug 50 µg/ml. Figur 5 zeigt die relativen Ausbeuten an aktiver Proteinase K in Abhängigkeit von der Konzentration des gewählten Pufferzusatzes.

### Beispiel 8:

### Aktivierung der naturierten pro-Proteinase K

Nach der Naturierung von pro-Proteinase K nach dem erfindungsgemäßen Verfahren läßt sich keine oder nur sehr geringe Aktivität feststellen. Durch chromatographiesche Methoden und SDS-PAGE läßt sich zeigen, daß reife Proteinase K bereits verhanden ist, aber noch in einem Komplex mit dem Propeptid assoziiert ist. Dieser läßt sich in einem hier als Aktivierung bezeichneten Verfahren, der auch Gegenstand der Erfindung ist, abtrennen.

In diesem Beispiel wurde dazu zu dem Faltungsansatz SDS in einer Konzentration von 2% (v/v) gegeben und anschließend die Faltungsadditive und das SDS durch Dialyse entfernt. Alternativ konnte SDS auch nach Entfernung der Additive durch Dialyse zugegeben werden. In allen Fällen konnte volle Aktivität der Proteinase K nachgewiesen werden.

### Beispiel 9:

### Charakterisierung des Faltungsproduktes

Die nach dem erfindungsgemäßen Verfahren naturierte und aktivierte Proteinase K wurde mit verschiedenen Methoden weiter charakterisiert:

### a) Reinheitsanalyse und Molekulargewichtsbestimmung mit SDS-Polyacrylamid-Gelelektrophorese

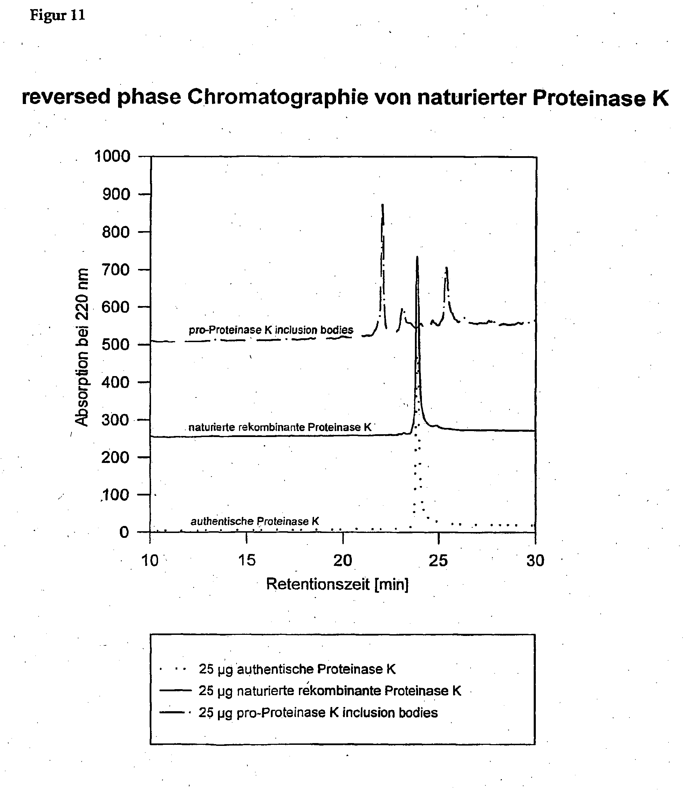

Auf einem 12,5%igen SDS Polyacrylamidgel wurden Aliquots von verschiedenen Schritten des Naturierungsprozesses sowie das Endprodukt, die gefaltete und aktivierte rekombinante Proteinase K aufgetragen. Die Proben enthielten jeweils 10 mM DTT oder 1% (v/v) 2-Mercaptoethanol. Die nach dem erfindungsgemäßen Verfahren hergestellte rekombinante Proteinase K zeigt keine nennenswerten Verunreinigungen und läuft identisch mit der authentischen Proteinase K bei einem apperenten Molekulargewicht von ca. 30 kDa (s. Figur 11).

### b) Reinheitsanalyse mit RP-HPLC

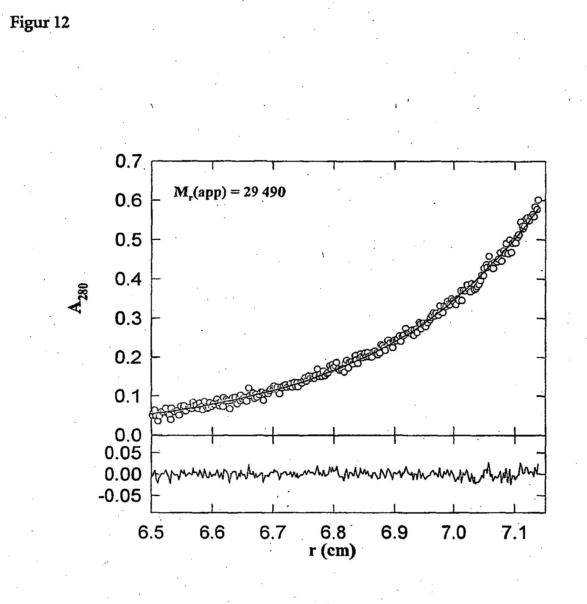

Die gefaltete und aktivierte Proteinase K sowie die authentische Proteinase K aus T. album und die pro-Proteinase K inclusion bodies wurden mittels reversed phase HPLC analysiert. Es wurde eine Vydac C4 Säule mit den Dimensionen 15 cm x 4,6 mm Durchmesser verwendet. Die Proben wurden mit einem Acetonitril Gradienten von 0% bis 80% in 0,1% TFA eluiert. Das Faltungsprodukt zeigt identische Laufeigenschaften wie die als Standard verwendete authentische Proteinase K (siehe Figur 12).

### c) Analytische Ultrazentrifugation

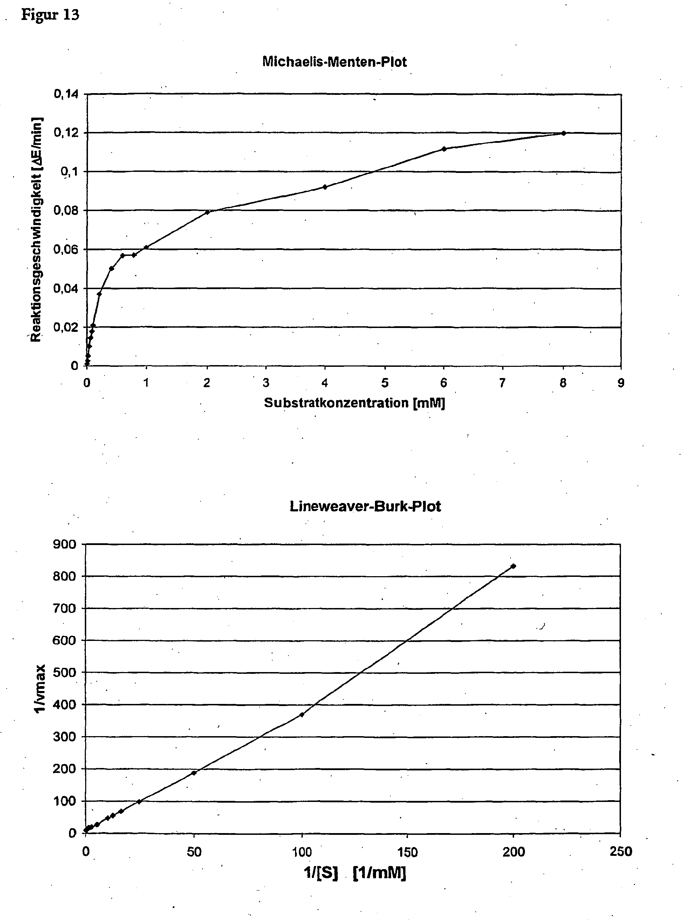

Um zu analysieren, ob die renaturierte und prozessierte Proteinase K in monomerer Form ohne Propeptid vorliegt, wurde das Protein mittels analytischer Ultrazentrifugation untersucht. Das ermittelte Molekulargewicht von 29 490 Da entspricht innerhalb der Fehlergrenzen dieser Methode der Masse der monomeren maturen Proteinase K (s. Figur 13). Somit konnte nachgewiesen werden, daß das Propeptid durch Aktivierung der Proteinase K quantitativ abgespalten wurde.

### d) N-terminale Sequenzanalyse

Um zu überprüfen, ob die Spaltung des Propetides an der korrekten Spaltstelle erfolgt, wurde die naturierte und aktivierte rekombinante Proteinase K einer Sequenzanalyse unterworfen. Dazu wurde das Faltungsprodukt wie in Beispiel 9 b) durch RP-HPLC entsalzt und die ersten 6 Reste durch N-terminale Sequenzierung untersucht. Das Ergebnis (AAQTNA) stimmt mit dem authentischen N-terminus der reifen Proteinase K überein.

### e) Aktivität und Kₘ-Wert

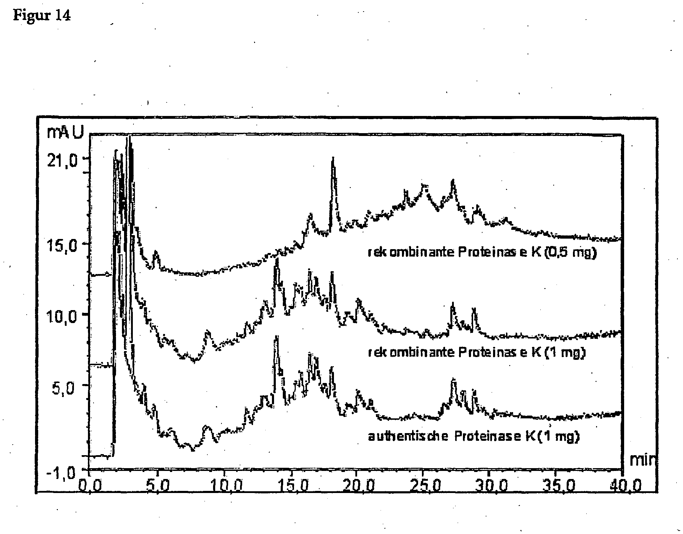

Der Kₘ-Wert der gefalteten und aktivierten Proteinase K wurde mit dem der authentischen Proteinase K verglichen. Als Substrat wurde das Tetrapeptid Suc-Ala-Ala-Pro-Phe-pNA verwendet. Der Test wurde in 2,0 ml 50 mM Tris, pH 8,5 mit 1 mM CaCl₂ bei 25°C durchgeführt. Die Hydrolyse des Peptids wurde spektroskopisch bei 410 nm verfolgt. Für die rekombinante Proteinase K wurde ein Kₘ-Wert von 0,16 mM in guter Übereinstimmung mit dem Kₘ-Wert der authentischen Proteinase K gefunden (s. Figur 14).

### f) Abbaumuster von Blutserumproteinen

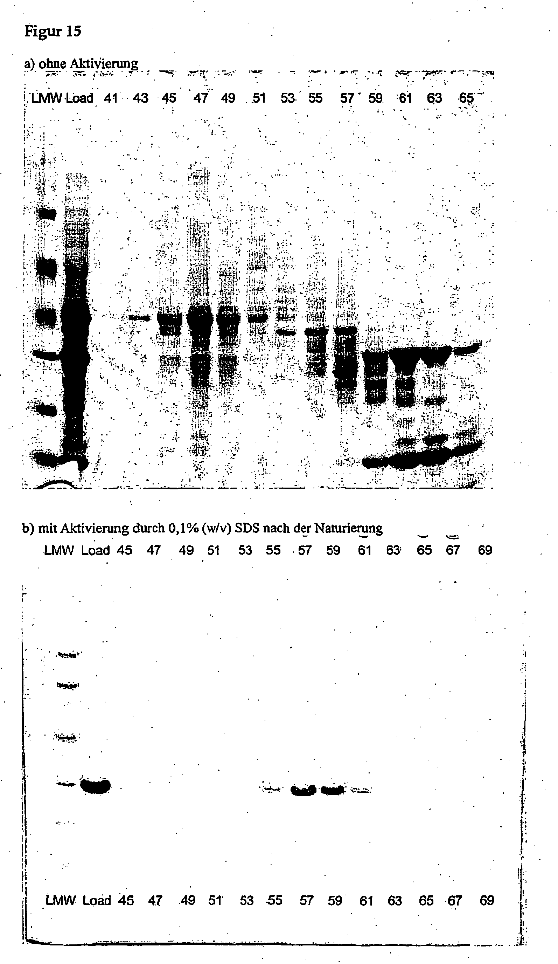

In einem weiteren Test zur Charakterisierung der Aktivität wurde das Spaltungsmuster von Blutserumproteinen untersucht. Dazu wurde eine definierte Menge an Blutserumproteinen mit 1 µg rekombinanter Proteinase K oder der gleichen Menge authentischer Proteinase K verdaut Das Spaltungsmuster wurde mittels RP-HPLC unter identischen Bedingungen wie in Beispiel 9 b) analysiert. In Figur 15 erkennt man, daß die rekombinante und die authentische Proteinase K zu einem identischen Abbaumuster führen.

### Beispiel 10:

### Aufreinigung des Faltungsprodukts

Die nach dem erfindungsgemäßen Verfahren naturierte rekombinante Proproteinase K wurde durch Gelfiltration gereinigt. Nach der Naturierung wie in Figur 11 beschrieben wurde die aufkonzentrierte Naturierungslösung im ersten Lauf ohne vorherige Aktivierung, im zweiten Lauf mit vorheriger Aktivierung mittels 0,15% (w/v) SDS (30 min, 4°C) auf einer Superdex 75 pg getrennt. Als Laufpuffer wurde 100 mM Tris/HCl,150 mM NaCl pH 8,75 (4°C) verwendet. Das Auftragsvolumen betrug 10 ml bei einem Säulenvolumen von 1200 ml und einer Flußrate von 5 ml/min. Nach Beendigung des Auftrags wurden 14 ml-Fraktionen gesammelt. Aliquots der Fraktionen wurden mit Trichloressigsäure gefällt, gewaschen und auf in Lämmli-Probenpuffer mit 10 mM DTT aufgenommen. Die Proben wurden auf ein 12,5%iges SDS Polyacrylamidgel aufgetragen, das nach dem Lauf mit Coomassie Blau R250 gefärbt wurde.

Bei dem ersten Lauf ohne Aktivierung sieht man in einem ersten Peak nicht prozessierte rekombinante Proproteinase K, die wahrscheinlich in Form von Mikroaggregaten im Ausschlußvolumen läuft. In einem zweiten Peak erkennt man prozessierte rekombinante Proteinase K, die mit dem Propeptid koeluiert, das nicht-kovalent gebunden ist und als Inhibitor wirkt. Deshalb konnte ohne weitere Aktivierung keine Aktivität festgestellt werden. Erst nach Zugabe von SDS zu den Fraktionen zeigt der zweite Peak deutliche Proteinase K Aktivität (nicht gezeigt).

Der zweite Lauf, bei dem die gefaltete rekombinante Proteinase K vorher mit SDS aktiviert wurde, zeigt nur einen Peak, der nach einem identischen Volumen wie die Proteinase K unter gleichen Bedingungen eluiert (nicht gezeigt). Auf dem SDS-Gel erkennt man in diesem Peak saubere reife rekombinante Proteinase K ohne Propeptid. Alle Verunreinigungen und das Propeptid scheinen bereits im Auftrag von der aktivierten rekombinante Proteinase K verdaut worden zu sein. Die Fraktionen des Proteinase K Peaks zeigten erwartungsgemäß auch ohne weitere Aktivierung mit SDS Aktivität. Die so gereinigte rekombinante Proteinase K erscheint auf dem SDS-Gel (Figur 16) zu fast 100% rein und zeigt ein identisches Laufverhalten wie die authentische Proteinase K.

## Patentansprüche

1. Verfahren zur Naturierung denaturierter, zymogener Proteinase K, wobei die denaturierte, zymogene Proteinase K in einen Faltungspuffer überführt wird, **dadurch gekennzeichnet daß** der Faltungspuffer folgende Merkmale aufweist:
• A) pH-Wert des Puffers liegt im Bereich von 7,5 und 10,5,
• B) Anwesenheit von niedermolekularen Faltungshilfsstoffen,
• C) Anwesenheit eines Redox-Shuffling-Systems,
• D) Anwesenheit eines Komplexbildners in einer substöchiometrischen Konzentration zu anwesenden Ca²⁺-Ionen,
• E) Anwesenheit von SDS in einer Konzentration zwischen 0,1% und 2% [w/v], und wobei
das Verfahren bei einer Temperatur zwischen 0°C und 37°C durchgeführt wird.

2. Verfahren gemäß Anspruch 1, wobei es sich bei dem Redox-Shuming-System um gemischte Disulfide oder Thiosulfonate handelt.

3. Verfahren gemäß einem der Ansprüche 1 oder 2, wobei der Puffer einen pH- Wert von pH 8 bis pH 9 aufweist.

4. Verfahren gemäß einem der Ansprüche 1 bis 3, wobei das Verfahren bei einer Temperatur zwischen 0°C und 25°C durchgeführt wird.

5. Verfahren gemäß einem der Ansprüche 1 bis 4, wobei während der Naturierung Denaturierungsmittel in einer Konzentration von kleiner als 50 mM anwesend sind.

6. Verfahren gemäß einem der Ansprüche 1 bis 5 wobei die niedermolekularen Faltungshilfsstoffe ausgewählt werden aus der folgenden Gruppe niedermolekularer Verbindungen und sowohl allein als auch in Mischungen zugesetzt werden können:
- L-Arginin in einer Konzentration von 0,5 M bis 2,0 M
- Tris in einer Konzentration von 0,5 M bis 2,0 M
- Triethanolamin in einer Konzentration von 0,5 M bis 2,0M
- α-Cyclodextrin einer Konzentration von 60 mM bis 120 mM.

7. Verfahren gemäß einem der Ansprüche 1 bis 6, wobei anwesende Ca²⁺-Ionen in einer Konzentration von 1 bis 20 mM vorliegen.

8. Verfahren gemäß einem der Ansprüche 1 bis 7, wobei die denaturierte zymogene Proteinase K unter Verringerung der Konzentration möglicherweise anwesender Denaturierungsmittel in den Faltungspuffer überführt wird.

9. Faltungspuffer, der durch folgende Merkmale **gekennzeichnet** ist:
• A) pH-Wert des Puffers liegt im Bereich von 7,5 und 10,5,
• B) Anwesenheit von niedermolekularen Faltungshilfsstoffen,
• C) Anwesenheit eines Redox-Shuffling-Systems,
• D) Anwesenheit eines Komplexbildners in einer substöchiometrischen Konzentration zu anwesenden Ca²⁺-Ionen,
• E) Anwesenheit von SDS in einer Konzentration zwischen 0,1% und 2% [w/v],
• F) Proteinase K.

10. Faltungspuffer gemäß Anspruch 9 wobei der Puffer einen pH- Wert von pH 8 bis pH 9 aufweist und wobei es sich bei dem Redox-Shuffling-System um gemischte Disulfide oder Thiosulfonate handelt.

11. Verwendung eines Puffers mit den Merkmalen
• A) pH-Wert des Puffers liegt im Bereich von 7,5 und 10,5,
• B) Anwesenheit von niedermolekularen Faltungshilfsstoffen,
• C) Anwesenheit eines Redox-Shuffling-Systems,
• D) Anwesenheit eines Komplexbildners in einer substöchiometrischen Konzentration zu anwesenden Ca²⁺-Ionen,
• E) Anwesenheit von SDS in einer Konzentration zwischen 0,1% und 2% [w/v], zur Faltung und Aktivierung denaturierter zymogener Proteinase K.

## Claims

1. Method for the naturation of denatured zymogenic proteinase K in which the denatured zymogenic proteinase K is transferred to a folding buffer, **characterized in that** the folding buffer has the following characteristics:
• A) pH of the buffer is in the range of 7.5 to 10.5,
• B) presence of low-molecular weight substances which aid folding,
• C) presence of a redox shuffling system,
• D) presence of a complexing agent at a substoichiometric concentration relative to the Ca²⁺ ions that are present,
• E) presence of SDS at a concentration between 0.1 % and 2 % [w/v], and wherein
the method is carried out at a temperature between 0°C and 37°C.

2. Method according to claim 1, wherein the redox shuffling system comprises mixed disulfides or thiosulfonates.

3. Method according to one of the claims 1 or 2, wherein the buffer has a pH of pH8topH9.

4. Method according to one of the claims 1 to 3, wherein the method is carried out at a temperature between 0°C and 25°C.

5. Method according to one of the claims 1 to 4, wherein denaturing agents are present during the naturation at a concentration of less than 50 mM.

6. Method according to one of the claims 1 to 5, wherein the low-molecular weight substances that aid folding are selected from the following group of low-molecular weight compounds and can be added alone or as mixtures:
- L-arginine at a concentration of 0.5 to 2.0 M,
- Tris at a concentration of 0.5 M to 2.0 M,
- triethanolamine at a concentration of 0.5 M to 2.0 M,
- α-cyclodextrin at a concentration of 60 mM to 120 mM.

7. Method according to one of the claims 1 to 6, wherein the Ca²⁺ ions that are present are present at a concentration of 1 to 20 mM.

8. Method according to one of the claims 1 to 7, wherein the denatured zymogenic proteinase K is transferred to the folding buffer while reducing the concentration of denaturing agents that may be present.

9. Folding buffer which is **characterized by** the following features:
• A) pH of the buffer is in the range of 7.5 to 10.5,
• B) presence of low-molecular weight substances which aid folding,
• C) presence of a redox shuffling system,
• D) presence of a complexing agent at a substoichiometric concentration relative to the Ca²⁺ ions that are present,
• E) presence of SDS at a concentration between 0.1 % and 2 % [w/v],
• F) proteinase K.

10. Folding buffer according to claim 9, wherein the buffer has a pH of pH 8 to pH 9 and wherein the redox shuffling system comprises mixed disulfides or thiosulfonates.

11. Use of a buffer having the following characteristics:
• A) pH of the buffer is in the range of 7.5 to 10.5,
• B) presence of low-molecular weight substances which aid folding,
• C) presence of a redox shuffling system,
• D) presence of a complexing agent at a substoichiometric concentration relative to the Ca²⁺ ions that are present,
• E) presence of SDS at a concentration between 0.1 % and 2 % [w/v] for folding and activating denatured zymogenic proteinase K.

## Revendications

1. Procédé pour la naturation de la protéinase K zymogène, dénaturée, la protéinase K zymogène, dénaturée étant transférée dans un tampon de pliage, **caractérisé en ce que** le tampon de pliage présente les caractéristiques suivantes :
• A) la valeur de pH du tampon se situe dans la plage de 7,5 à 10,5,
• B) la présence d'adjuvants de pliage à bas poids moléculaire,
• C) la présence d'un système de réarrangement redox,
• D) la présence d'un formateur de complexe en une concentration sous-stoechiométrique par rapport à celle des ions Ca²⁺ présents,
• E) la présence de SDS en une concentration entre 0,1 % et 2 % [en poids/volume], et
le procédé étant mis en oeuvre à une température entre 0 °C et 37 °C.

2. Procédé selon la revendication 1, dans lequel, en ce qui concerne le système de réarrangement redox, il s'agit de disulfures ou de thiosulfonates mixtes.

3. Procédé selon l'une quelconque des revendications 1 ou 2, dans lequel le tampon présente une valeur de pH de 8 à 9.

4. Procédé selon l'une quelconque des revendications 1 à 3, dans lequel le procédé est mis en oeuvre à une température entre 0 °C et 25 °C.

5. Procédé selon l'une quelconque des revendications 1 à 4, dans lequel, au cours de la naturation, des agents de dénaturation sont présents en une concentration inférieure à 50 mM.

6. Procédé selon l'une quelconque des revendications 1 à 5, dans lequel les adjuvants de pliage à bas poids moléculaire sont choisis parmi le groupe suivant de composés à bas poids moléculaire et peuvent être ajoutés aussi bien seuls qu'en mélange :
- de la L-arginine en une concentration de 0,5 M à 2,0 M,
- du Tris en une concentration de 0,5 M à 2,0 M,
- de la triéthanolamine en une concentration de 0,5 M à 2,0 M,
- de l'α-cyclodextrine en une concentration de 60 mM à 120 mM.

7. Procédé selon l'une quelconque des revendications 1 à 6, dans lequel des ions Ca²⁺ présents le sont en une concentration de 1 à 20 mM.

8. Procédé selon l'une quelconque des revendications 1 à. 7, dans lequel la protéinase K zymogène, dénaturée est transférée dans le tampon de pliage, tout en réduisant la concentration des agents de dénaturation éventuellement présents.

9. Tampon de pliage, qui se **caractérise par** les particularités suivantes :
• A) la valeur de pH du tampon se situe dans la plage de 7,5 à 10,5,
• B) la présence d'adjuvants de pliage à bas poids moléculaire,
• C) la présence d'un système de réarrangement redox,
• D) la présence d'un formateur de complexe en une concentration sous-stoechiométrique par rapport à celle des ions Ca²⁺ présents,
• E) la présence de SDS en une concentration entre 0,1 % et 2 % [en poids/volume],
• F) la protéinase K.

10. Tampon de pliage selon la revendication 9, dans lequel le tampon présent une valeur de pH de 8 à 9 et dans lequel, en ce qui concerne le système de réarrangement redox, il s'agit de disulfures ou de thiosulfonates mixtes.

11. Utilisation d'un tampon comprenant les caractéristiques :
• A) la valeur de pH du tampon se situe dans la plage de 7,5 à 10,5,
• B) la présence d'adjuvants de pliage à bas poids moléculaire,
• C) la présence d'un système de réarrangement redox,
• D) la présence d'un formateur de complexe en une concentration sous-stoechiométrique par rapport à celle des ions Ca²⁺ présents,
• E) la présence de SDS en une concentration entre 0,1 % et 2 % [en poids/volume],
pour le pliage et l'activation de la protéinase K zymogène, dénaturée.
